# EUROPÄISCHE PATENTSCHRIFT

(11) **EP 0 249 096 B1**
(45) Veröffentlichungstag und Bekanntmachung des Hinweises auf die Patenterteilung: **11.01.1995**
(21) Anmeldenummer: 87107774.9
(22) Anmeldetag: 28.05.1987
(51) Int. Cl.: C07K 5/02, A61K 31/16, A61K 31/395, A61K 38/55, C07D 213/56, C07D 239/42, C07D 241/20, C07D 257/04, C07K 1/00

(54) **Reninhemmende Aminosäurederivate**
Renin-inhibiting amino acid derivatives
Dérivés d'acides aminés inhibant la rénine

(30) Priorität: 10.06.1986 DE 19863619508; 11.11.1986 DE 19863638425
(43) Veröffentlichungstag der Anmeldung: 16.12.1987
(73) Patentinhaber: MERCK PATENT GmbH, D-64271 Darmstadt (DE)
(72) Erfinder: Raddatz, Peter, Dr., D-6100 Darmstadt (DE); Sombroek, Johannes, Dr., D-6100 Darmstadt (DE); Gante, Joachim, Dr., D-6100 Darmstadt (DE); Schmitges, Claus J. Dr., D-6114 Gross-Umstadt (DE); Minck, Klaus Otto, Dr., D-6105 Ober-Ramstadt (DE); Jonczyk, Alfred, Dr., D-6100 Darmstadt (DE); Hölzemann, Günter, Dr., D-6104 Seeheim 1 (DE)

(56) Entgegenhaltungen:
- EP-0 161 588
- PEPTIDES, STRUCTURE AND FUNCTION, Proceedings of the ninth American Peptide Symposium, page 747, edited by C.M. Deber, Pierce Chemical Co., Rockford, Illinois, US; J. BOGER et al.: "Renin inhibitors, tripeptide and tetrapeptide competitive inhibitors containing a novel analog of statine"
- PEPTIDES, STRUCTURE AND FUNCTION, Proceedings of the 9th American PeptideSymposium, C.M. Deber (ed.), Pierce Chemical Co., Rockford, IL (US); J. BOGERet al., p. 747

## Beschreibung

Die Erfindung betrifft neue Aminosäurederivate der Formel I

X-(NR¹-CHR²-CO)ₖ-Z-(CH₂)ₘ-CO-NR³-CHR⁴-CR⁵-(CHR⁶)ₙ-CO-E-Q-Y I

worin
- X: H, R⁷-O-CₘH₂ₘ-CO-, R⁷-CₘH₂ₘ-O-CO-, R⁷-CₘH₂ₘ-CO-, (R⁷-CₘH₂ₘ)-L(R⁸-CₚH₂ₚ)-CᵣH₂ᵣ-CO-, oder (R⁷-CᵣH₂ₘ-(T)ₓ-CO-CᵣH₂ᵣ)-L(R⁸-CₚH₂ₚ)-CO-,
- Z: -CH₂-, -NR⁹- oder -CH₂-S-,
- E: 1 oder 2 peptidartig miteinander verbundene Aminosäurereste ausgewählt aus der Gruppe bestehend aus Ile, Leu, Phe und Val,
- T: O oder NH,
- Q-Y: OH, OA, NH-CₜH₂ₜ-R¹⁰ oder NHC_{w}H_{2w}-(CR¹¹)ₛ-CₜH₂ₜ-R¹⁰,
- R¹ und R⁹: jeweils H oder A,
- R²: A, Ar-alkyl, Cyclohexymethyl, 1-oder 2-Dekalylmethyl oder Hetalkyl,
- R³: H,
- R⁴: A, Cycloalkylalkyl mit 4-11 C-Atomen oder Benzyl,
- R⁵: (H,OH) oder (H,NH₂),
- -(CHR⁶)ₙ-: -CH₂-, -CH₂CH₂- oder -CH₂CHA-,
- R⁷, und R10: jeweils H, A, Ar oder Het,
- R⁸: Benzyl oder 1- oder 2-Naphthylmethyl,
- R¹¹: (H,OH),(H,NH₂) oder = O,
- L: CH oder N,
- k und x: jeweils 0 oder 1,
- m: 1,2 oder 3,
- p, r und t: jeweils 0, 1, 2, 3, 4 oder 5,
- s: 1 oder 2,
- w: 1, 2, 3, 4 oder 5,
- Ar: unsubstituiertes oder einfach durch A, AO, Hal oder Aminoalkyl mit 1-8 C-Atomen substituiertes Phenyl oder unsubstituiertes Naphthyl,
- Het: einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, HO, Carbonylsauerstoff, H₂N, AOOC, H₂NCO, Hydroxyalkyl und/oder Aminoalkyl mit jeweils 1-8 C-Atomen substituiert sein kann und/oder dessen S-Heteroatom auch oxydiert sein kann,
- Hal: F, Cl, Br oder J und
- A: Alkyl mit 1-8 C-Atomen bedeuten,
sowie deren Salze.

Ähnliche Verbindungen sind aus der EP-A-163237 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z. B. nach der Methode von F. Fyhrquist et al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z. B. Pepsin und Kathepsin D) sind in der Regel wesentlich höhere (etwa 100 bis 10 000mal so hohe) Konzentrationen dieser Verbindungen notwendig.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen. Solche diagnostische Tests können in der in der EP-A-77028 beschriebenen Weise durchgeführt werden.

Die vor- und nachstehend aufgeführten Abkürzungen von Aminosäureresten stehen für die Reste -NR-CR'R"-CO-(worin R, R' und R" die für jede Aminosäure bekannte spezifische Bedeutung hat) folgender Aminosäuren:
- Abu: 2-Aminobuttersäure
- Ala: Alanin
- Cal: 3-Cyclohexylalanin
- Gly: Glycin
- His: Histidin
- Ile: Isoleucin
- Leu: Leucin
- Met: Methionin
- *α*-Nal: 3-*α*-Naphthyl-alanin
- *β*-Nal: 3-*β*-Naphthyl-alanin
- Nle: Norleucin
- Phe: Phenylalanin
- Sar: Sarcosin (N-Methylglycin)
- Trp: Tryptophan
- Tyr: Tyrosin
- Val: Valin.
Ferner bedeutet nachstehend:
- BOC: tert.-Butoxycarbonyl
- imi-BOM: Benzyloxymethyl in 1-Stellung des Imidazolrings
- CBZ: Benzyloxycarbonyl
- DNP: 2,4-Dinitrophenyl
- imi-DNP: 2,4-Dinitrophenyl in 1-Stellung des Imidazolrings
- FMOC: 9-Fluorenylmethoxycarbonyl
- IPOC: Isopropoxycarbonyl
- OMe: Methylester
- OEt: Ethylester
- POA: Phenoxyacetyl
- DCCI: Dicyclohexylcarbodiimid
- HOBt: 1-Hydroxybenzotriazol.

Sofern die vorstehend genannten Aminosäuren in mehreren enantiomeren Formen auftreten können, so sind vor- und nachstehend, z. B. als Bestandteil der Verbindungen der Formel I, alle diese Formen und auch ihre Gemische (z. B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt
oder daß man eine Carbonsäure der Formel II

X-(NR¹-CHR²-CO)ₖ-G¹-OH II

worin
G¹
   (a) fehlt,
   (b) -Z-(CH₂)ₘ-CO-,
   (c) -Z-(CH₂)ₘ-CO-W-,
   (d) -Z-(CH₂)ₘ-CO-W-E¹-,
   (e) -Z-(CH₂)ₘ-CO-W-E- und
W -NR³-CHR⁴-CR⁵-(CHR⁶)ₙ-CO- bedeuten oder eines ihrer reaktionsfähiger Derivate mit einer Verbindung der Formel III

   H-G² III

   worin
G²
   (a) -Z¹-(CH₂)ₘ-CO-W-E-Q-Y,
   (b) -W-E-Q-Y,
   (c) -E-Q-Y,
   (d) -E²-Q-Y,
   (e) -Q-Y,
Z¹ -O- oder -NR⁹- und
E¹ + E² zusammen E bedeuten
oder einem ihrer reaktionsfähigen Derivate umsetzt,
und/oder daß man gegebenenfalls in einer Verbindung der Formel I eine funktionell abgewandelte Amino- und/oder Hydroxygruppe duch Behandeln mit solvolysierenden oder hydrogenolysierenden Mitteln in Freiheit setzt und/oder eine freie Aminooder Hydroxygruppe acyliert und/oder eine Ketogruppe zu einer CHOH-Gruppe reduziert oder zu einer CH(NH₂)-Gruppe reduktiv aminiert und/oder eine Verbindung der Formel I durch Behandeln mit einer Säure in eines ihrer Salze überführt.

Vor- und nachstehend haben die Reste bzw. Parameter X, Z, E, Q, T, Y, R¹ bis R¹¹, L, k, m, p, r, s, t, w, x, Ar, Het, Hal, A, G¹, G², W, Z¹, E¹ und E² die bei den Formeln I, II oder III angegebenen Bedeutungen, falls nicht ausdrücklich etwas anderes angegeben ist.

In den vorstehenden Formeln hat A 1 - 8, vorzugsweise 1,2,3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z. B. 1-, 2- oder 3-Methylcyclopentyl, 1-, 2-, 3- oder 4-Methylcyclohexyl.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z. B. 1-, 2- oder 3-Methylcyclopentylmethyl, 1-, 2-, 3- oder 4-Methylcyclohexylmethyl.

Hal bedeutet vorzugsweise F, Cl oder Br, aber auch J.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Aminomethylphenyl, 1- oder 2-Naphthyl.

Dementsprechend bedeutet Ar-alkyl vorzugsweise Benzyl, 1- oder 2-Phenylethyl, o-, m- oder p-Methylbenzyl, 1- oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1-oder 2-o-, -m- oder -p-Chlorphenylethyl, o-, m-oder p-Brombenzyl, 1- oder 2-o-, -m- oder -p-Bromphenylethyl, o-, m- oder p-Jodbenzyl, 1- oder 2-o-, -m- oder -p-Jodphenylethyl, o-, m- oder p-Aminomethylbenzyl, 1- oder 2-Naphthylmethyl.

Het ist vorzugsweise 2- oder 3-Furyl, 2- oder 3-Thienyl, 1-, 2- oder 3-Pyrryl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidyl, weiterhin bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2-oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3-oder 4-Pyridazinyl, Pyrazinyl, 2-, 3-, 4-, 5-, 6 oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7-, oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Chinnolyl, 2-, 4-, 5-, 6-, 7- oder 8-Chinazolyl. Die heterocyclischen Reste können auch teilweise oder vollständig hydriert sein. Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, Tetrahydro-2- oder -3-thienyl, 2,3-Dihydro-1-, -2-, -3-, -4- oder -5-pyrryl, 2,5-Dihydro-1-, -2-, -3-, -4- oder -5-pyrryl, 1-, 2- oder 3-Pyrrolidinyl, Tetrahydro-1-, -2-oder -4-imidazolyl, 2,3-Dihydro-1-, -2-, -3-, -4- oder 5-pyrazolyl, 2,5-Dihydro-1-, -2-, -3-, -4- oder 5-pyrazolyl, Tetrahydro-1-, -3-, oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidyl, 1-, 2- oder 3-Piperazinyl, 1,2,3,4-Tetrahydro-1-, -2-, -3-, -4-, -5-, -6-, -7-, oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann also bevorzugt auch bedeuten: 2-Amino-4-thiazolyl, 4-Carbamoyl-2-thiazolyl, 4-(2-Aminoethyl)-2-thiazolyl, 2-Amino-5,6-dimethyl-3-pyrazinyl, 4-Carbamoylpiperidino, ferner z. B. 3-, 4- oder 5-Methyl-2-furyl, 2-, 4-oder 5-Methyl-3-furyl, 2,4-Dimethyl-3-furyl, 3-, 4-oder 5-Methyl-2-thienyl, 2-, 4- oder 5-Methyl-3-thienyl, 3-Methyl-5-tert.-butyl-2-thienyl, 1-, 3-, 4- oder 5-Methyl-2-pyrryl, 3,5-Dimethyl-4-ethyl-2-pyrryl, 4-Methyl-5-pyrazolyl, 4- oder 5-Methyl-2-thiazolyl, 2-oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 2-Hydroxy-3-, -4-, -5-oder -6-pyridyl (= 1H-2-Pyridon-3-, -4-, -5- oder -6-yl), 2-Methyl-3-hydroxy-4-hydroxymethyl-5-pyridyl, 2-Hydroxy-4-amino-6-methyl-3-pyridyl, 5- oder 6-Methyl-4-pyrimidyl, 2,6-Dihydroxy-4-pyrimidyl, 2-Methyl-4-amino-5-pyrimidyl, 3-Methyl-2-benzofuryl, 2-Ethyl-3-benzofuryl, 7-Methyl-2-benzothienyl, 1-, 2-, 4-, 5-, 6- oder 7-Methyl-3-indolyl, 1-Methyl-5-oder -6-benzimidazolyl, 1-Ethyl-5- oder -6-benzimidazolyl, 3-, 4-, 5-, 6-, 7- oder 8-Hydroxy-2-chinolyl.

R¹ und R⁹ bedeuten vorzugsweise H oder Methyl, ferner Ethyl, Propyl, Isopropyl, Butyl oder Isobutyl.

R² und R⁴ bedeuten vorzugsweise Cyclohexylmethyl, ferner bevorzugt A, insbesondere Methyl, Ethyl, Propyl, Isopropyl, Butyl, Isobutyl, sek.-Butyl, Pentyl, Isopentyl (3-Methylbutyl) oder 2-Methylbutyl, Benzyl oder 2-Cyclohexylethyl,

R⁵ ist vorzugsweise (H, OH).

R⁷ bedeutet vorzugsweise A, insbesondere Methyl, Ethyl, Propyl, Isopropyl, Butyl, Isobutyl oder tert.-Butyl, weiterhin vorzugsweise oder Phenyl R⁸ ist vorzugsweise Benzyl.

R¹⁰ ist vorzugsweise H, A oder Het.

R¹¹ ist vorzugsweise = O.

E bedeutet vorzugsweise einen der angegebenen Aminosäurereste, insbesondere Ile.

Die Parameter m, p und r sind vorzugsweise 0, 1 oder 2; s ist vorzugsweise 1. Die Gruppen CₜH₂ₜ und C_{w}H_{2w} sind vorzugsweise jeweils -CH₂-, -(CH₂)₂-, -CH(CH₃)-, -CH(Isobutyl)- oder -CH(sek.-Butyl).

X bedeutet vorzugsweise H, POA, Alkoxycarbonyl wie IPOC oder BOG; Ar-alkoxycarbonyl wie CBZ; Alkanoyl mit vorzugsweise 2-7 C-Atomen wie Acetyl, Propionyl, Butyryl, Isobutyryl, Hexanoyl, Heptanoyl, 2-Propylvaleryl (= Dipropylacetyl); Ar-CO wie Benzoyl; Ar-alkanoyl wie Phenylacetyl, 2-oder 3-Phenylpropionyl, 4-Phenylbutyryl, 2-Benzylbutyryl, 2-Benzyl-hexanoyl, 2-Benzyl-5-methylhexanoyl, 2-Benzyl-heptanoyl; Di-Ar-alkanoyl wie 2-Benzyl-3-phenylpropionyl, 2-Benzyl-4-phenylbutyryl, 2-(2-Phenylethyl)-4-phenylbutyryl, 2-(1- oder 2-Naphthylmethyl)-4-phenylbutyryl, 2- oder 3-o-, -m-oder -p-Fluorphenylpropionyl, 2- oder 3-o-, -m-oder -p-Chlorphenylpropionyl; Cycloalkylalkanoyl wie Cyclohexylacetyl, 2-oder 3-Cyclohexylpropionyl; FMOC; heterocyclisch substituiertes Arylalkanoyl wie 2-Benzyl-4-(2-, -3- oder -4-pyridyl)-butyryl, 2-Benzyl-4-(2-oxopyrrolidino)-butyryl; N-substituiertes Carbamoyl wie N-Benzylcarbamoyl, N-(2-, 3-oder 4-Pyridylmethyl)-carbamoyl; N,N-disubstituiertes Carbamoyl wie N,N-Dibenzyl-carbamoyl, N-Benzyl-N-2-phenylethyl-carbamoyl, N-Benzyl-N-butyl-carbamoyl, N,N-Bis-2-phenylethyl-carbamoyl, N-2-Morpholinoethyl-N-1-naphthylmethyl-carbamoyl, Morpholinocarbonyl; durch Alkoxycarbonyl substituiertes Arylalkanoyl wie 2-Benzyl-3-ethoxy carbonylpropionyl, 2-(1-Naphthylmethyl)-3-ethoxycarbonyl-propionyl; durch substituiertes Carbamoyl substituiertes Arylalkanoyl wie 2-Benzyl-3-(N-isopropyl-carbamoyl)-propionyl, 2-(1-Naphthylmethyl)-3-(N-isopropyl-carbamoyl)-propionyl, 2-(1-Naphthylmethyl)-3-(N-2-phenylethylcarbamoyl)-propionyl, 2-Benzyl-3-piperidinocarbonylpropionyl, 2-Benzyl-3-morpholinocarbonyl-propionyl, 2-(1-Naphthylmethyl)-3-morpholinocarbonyl-propionyl; durch Acyl substituiertes Arylalkanoyl wie 2-Benzyl-4-oxo-hexanoyl, 2-Benzyl-4-oxo-5,5-dimethylhexanoyl, 2-Benzyl-4-oxo-6-methylheptanoyl, 2-Benzyl-4-oxo-4-phenylbutyryl, 2-Benzyl-4-oxo-4-(2-, -(3- oder -(4-pyridyl)-butyryl.

Besonders bevorzugte Reste X sind H und BOC, ferner POA, 4-Phenyl-butyryl, 2-Benzyl-butyryl, 2-Benzyl-hexanoyl, 2-Benzyl-heptanoyl, 2-Benzyl-3-phenylpropionyl, 2-Benzyl-4-phenylbutyryl, 2-(2-Phenylethyl)-4-phenylbutyryl, 2-(1-Naphthylmethyl)-4-phenylbutyryl, CBZ, N-Benzylcarbamoyl, N-(2-Pyridylmethyl)-carbamoyl, N,N-Dibenzylcarbamoyl, 2-(1-Naphthylmethyl)-3-ethoxycarbonyl-propionyl und 2-Benzyl-4-oxo-4-(2-pyridyl)-butyryl.

Die Gruppe -NR¹-CHR²-CO- bedeutet vorzugsweise Phe, ferner bevorzugt Cal, *α*Nal oder *β*Nal.

Z bedeutet vorzugsweise -NR⁹-, insbesondere -NH- oder -N(CH₃)-, ferner bevorzugt -CH₂- oder -CH₂-S-.

Dementsprechend bedeutet die Gruppe -Z-(CH₂)ₘ-CO- vorzugsweise Gly, ferner bevorzugt -CH₂CH₂CO-, -NH-CH₂CH₂-CO- oder -CH₂-S-CH₂-CO-.

Die Gruppe W bedeutet vorzugsweise -NH-CHR⁴-CHOH-CH₂-CO-, insbesondere -NH-CH-(Cyclohexylmethyl)-CHOH-CH₂-CO- ("AHCP", abgeleitet von 4-Amino-3-hydroxy-5-cyclohexylpentansäure), ferner -NH-CH(CH₂CH₂-Cyclohexyl)-CHOH-CH₂-CO- ("AHCH"; abgeleitet von 4-Amino-3-hydroxy-6-cyclohexyl-hexansäure), -NH-CH-(Isobutyl)-CHOH-CH₂-CO- ("Sta"; abgeleitet von Statin) oder -NH-CH(Benzyl)-CHOH-CH₂-CO ("AHPP"; abgeleitet von 4-Amino-3-hydroxy-5-phenylpentansäure). Die Gruppe W bedeutet ferner bevorzugt -NH-CHR⁴-CH(NH₂)-CH₂-CO-, insbesondere -NH-CH(Cyclohexylmethyl)-CH(NH₂)-CH₂-CO-("DACP"; abgeleitet von 3,4-Diamino-5-cyclohexyl-pentansäure), -NH-CH(CH₂CH₂-Cyclohexyl)-CH-(NH₂)-CH₂-CO-("DACH"; abgeleitet von 3,4-Diamino-6-cyclohexylhexansäure), -NH-CH(Isobutyl)-CH-(NH₂)-CH₂-CO- ("DAMH"; abgeleitet von 3,4-Diamino-6-methylheptansäure) oder -NH-CH(Benzyl)-CH(NH₂)-CH₂-CO- ("DAPP"; abgeleitet von 3,4-Di-amino-5-phenylpentansäure).

Die Gruppe W besitzt mindestens zwei chirale Zentren. Die Verbindungen der Formel I können daher in verschiedenen - optisch-inaktiven oder optisch-aktiven - Formen vorkommen. Die Formel I umschließt alle diese Formen. Falls W -NH-CHR⁴-CR⁵-CH₂-CO- und R⁵ (H, OH) oder (H, NH₂) bedeuten, sind die 3S-Hydroxy-4S-amino-Enantiomeren bzw. 3S,4S-Diamino-Enantiomeren bevorzugt. Wenn bei der Bezeichnung von Einzelsubstanzen nichts anderes angegeben ist, beziehen sich die Abkürzungen AHCP, AHCH, Sta, AHPP, DACP, DACH, DAMH und DAPP immer auf diese 3S,4S-Formen.

Eine besonders bevorzugte Bedeutung der Gruppe X-NR¹-CHR²-CO-Z-(CH₂)ₘ-CO-W- ist BOC-Phe-Gly-AHCP. Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis li ausgedrückt werden, die der Formel I (k = 0) entsprechen, worin jedoch
- in la: -Z-(CH₂)ₘ-CO- Gly, Sar oder -CH₂CH₂CO-bedeutet;
- in Ib: -Z-(CH₂)ₘ-CO- Gly bedeutet;
- in Ic: -NR³-CHR⁴-CR⁵-(CHR⁶)ₙ-CO- (= W)-AHCP, AHCH, Sta, AHPP, DACP, DACH, DAMH oder DAPP bedeutet;
- in Id: W AHCP bedeutet;
- in Ie: E Ile oder Leu bedeutet;
- in If: E Ile bedeutet,
- in Ig: -Q-Y -NH₂, -NH-CH₂-(3-pyridyl), -NH-CH₂-(4-amino-2-methyl-5-pyrimidinyl) oder -NH-CH₂-(2-amino-5,6-dimethyl-3-pyrazinyl) bedeutet;
- in Ih: -Z-(CH₂)ₘ-CO- Gly oder -CH₂CH₂CO-,
W AHCP,
E Ile oder Leu und
-Q-Y -NH₂, -NH-CH₂-(3-pyridyl), -NH-CH₂-(4-amino-2-methyl-5-pyrimidinyl) oder -NH-CH₂-(2-amino-5,6-dimethyl-3-pyrazinyl) bedeuten;
- in Ii: -Z-(CH₂)ₘ-CO- Gly,
W AHCP,
E Ile und
-Q-Y -NH₂, -NH-CH₂-(3-pyridyl), -NH-CH₂-(4-amino-2-methyl-5-pyrimidinyl) oder -NH-CH₂-(2-amino-5,6-dimethyl-3-pyrazinyl) bedeuten.

Ferner sind bevorzugt Verbindungen der Formeln I' sowie la' bis Ii', die den Formeln I (k = 0) sowie la bis li entsprechen, worin jedoch
- X: H, BOC, CBZ, POA, Hexanoyl, Heptanoyl, 2-Propylvaleryl, 3-Phenylpropionyl, 4-Phe-nylbutyryl, 2-Benzylbutyryl, 2-Benzylhexanoyl, 2-Benzylheptanoyl, 2-Benzyl-4-phenyl-butyryl, 2-Benzyl-4-(4-pyridyl)-butyryl, 2-Benzyl-4-(2-oxopyrrolidino)-butyryl, N-Benzyl-carbamoyl, N-2-Pyridylmethylcarbamoyl, N,N-Dibenzyl-carbamoyl, N-Benzyl-N-butyl-carbamoyl, N-Benzyl-N-2-phenylethyl-carbamoyl, N,N-Bis-2-phenylethyl-carbamoyl, N-2-Morpholinoethyl-N-1-naphthylmethyl-carbamoyl, 2-Benzyl-3-ethoxycarbonyl-propionyl, 2-(1-Naphthylmethyl)-3-ethoxycarbonyl-propionyl, 2-(1-Naphthylmethyl)-3-(N-isopropyl-carbamoyl)-propionyl, 2-(1-Naphthylmethyl)-3-morpholinocarbonyl-propionyl, 2-Benzyl-4-oxo-hexanoyl, 2-Benzyl-4-oxo-5,5-dimethylhexanoyl, 2-Benzyl-4-oxo-6-methyl-heptanoyl, 2-Benzyl-4-oxo-4-phenyl-butyryl oder 2-Benzyl-4-oxo-4-(2-pyridyl)-butyryl bedeutet.

Ferner sind bevorzugt Verbindungen der Formeln I" sowie la" bis Ii" die den Formeln I (k = 0) sowie la bis li entsprechen, worin jedoch
- X: H, BOC, CBZ, POA, 2-Benzyl-heptanoyl, 2-Benzyl-4-(2-oxopyrrolidino)-butyryl, N,N-Dibenzyl-carbamoyl, oder N-Benzyl-N-2-phenylethyl-carbamoyl bedeutet.

Einige weitere bevorzugte Gruppen von Verbindungen können durch die folgenden Teilformeln Ik bis II' ausgedrückt werden, die der Formel I (k = 1) entsprechen, worin jedoch
- in Ik: X BOC bedeutet;
- in Il: -NR¹-CHR²-CO- Phe bedeutet;
- in Im: -Z-(CH₂)ₘ-CO- Gly bedeutet;
- in In: X-NR-CHR²-CO- BOC-Phe- bedeutet;
- in Io: -NR¹-CHR²-CO-Z-(CH₂)ₘ-CO -Phe-Gly- bedeutet;
- in Ip: X-NR¹-CHR²-CO-Z-(CH₂)ₘ-CO-BOC-Phe-Gly- bedeutet;
- in Iq: W AHCP bedeutet;
- in Ir: X-NR¹-CHR²-CO-Z-(CH₂)ₘ-CO-W-BOC-Phe-Gly-AHCP- bedeutet;
- in Is: E Ile bedeutet;
- in It: X-NR¹-CHR²-CO-Z-(CH₂)ₘ-CO-W-E-BOC-Phe-Gly-AHCP-Ile bedeutet;
- in lu: X-NR¹-CHR²-CO-Z-(CH₂)ₘ-CO-W-BOC-Phe-Gly-AHCP-,
E Ile und
Q-Y OH, OMe, NH₂, 3-Pyridylmethylamino, 4-Amino-2-methyl-5-pyrimidinylmethylamino oder 2-Amino-5,6-di-methyl-3-pyrazinylmethylamino bedeuten.

Die Verbindungen der Formel I und auch die Ausgangsstoffe zu ihrer Herstellung werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; ferner EP-A-45665, EP-A-77028, EP-A-77029, EP-A-81783) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

So können die Verbindungen der Formel I erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z. B. solche, die der Formel I entsprechen, aber an Stelle einer His-Gruppe eine N(im)-R¹⁴-His-Gruppe (worin R¹⁴ eine Aminoschutzgruppe bedeutet, z. B. BOM oder DNP) enthalten, oder solche der Formel X-(NR¹-CHR²-CO)ₖ-Z-(CH₂)ₘ-CO-NR³-CHR⁴-CH(NHR¹⁴)-(CHR⁶)ₙ-CO-E-Q-Y.

Ferner sind Ausgangsstoffe bevorzugt, die an Stelle des H-Atoms einer Hydroxygruppe eine Hydroxyschutzgruppe tragen, z. B. solche der Formel X-(NR¹-CHR²-CO)ₖ-Z-(CH₂)ₘ-CO-NR³-CHR⁴-CHOR¹⁵-(CHR⁶)ₙ-CO-E-Q-Y, worin R¹⁵ eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls duchgeführt worden ist. Typisch für solche Gruppen sind insbesondere unsubstituierte oder substituierte Acyl-, Aryl- (z. B. 2,4-Dinitrophenyl), Aralkoxymethyl- (z. B. Benzyloxymethyl) oder Aralkylgruppen (z. B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonylund vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind DNP, BOM, CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäureund Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingtje nach der benutzten Schutzgruppe - z. B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzoloder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran (THF) oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z. B. bevorzugt mit 40 %iger Trifluoressigsäure in Dichlormethan oder mit etwa 3 bis 5 n HCl in Dioxan bei 15-30° abgespalten werden, die FMOC-Gruppe mit einer etwa 5-bis 20%igen Lösung von Dimethylamin, Diethylamin oder Piperidin in DMF bei 15-30°. Eine Abspaltung der DNP-Gruppe gelingt z. B. auch mit einer etwa 3- bis 10%igen Lösung von 2-Mercaptoethanol in DMF/Wasser bei 15-30°.

Hydrogenolytisch entfernbare Schutzgruppen (z. B. BOM, CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5-bis 10%igem Pd-C in Methanol bei 20-30°.

Verbindungen der Formel I können auch durch direkte Peptidsynthese aus einer Carbonsäure- und einer Aminkomponente erhalten werden. Als Carbonsäurekomponenten eignen sich z. B. solche der Teilformeln (a) X-(NR¹-CHR²-CO)ₖ-OH, (b) X-(NR¹-CHR²-CO)ₖ-Z-(CH₂)ₘ-COOH, (c) X-(NR¹-CHR²-CO)-ₖ-Z-(CH₂)ₘ-CO-W-OH, (d) X-(NR¹-CHR²-CO)ₖ-Z-(CH₂)ₘ-CO-W-E¹-OH oder (e) X-(NR¹-CHR²-CO)ₖ-Z-(CH₂)ₘ-CO-W-E-OH, als Aminkomponenten solche der Teilformeln (a) H-Z¹-(CH₂)ₘ-CO-W-E-Q-Y, (b) H-W-E-Q-Y, (c) H-E-Q-Y, (d) H-E²-Q-Y oder (e) H-Q-Y. Im Fall (d) wird die Peptidbindung innerhalb der Gruppe E geknüpft, falls E aus zwei der angegebenen Aminosäurereste besteht; dabei wird eine Carbonsäure mit einem Amin der jeweils unter (d) angegebenen Formeln umgesetzt, wobei E¹ und E² jeweils einen der bei der Definition von E angegebenen Aminosäurereste bedeutet und E¹ + E² = E ist. Dabei arbeitet man zweckmäßig nach üblichen Methoden der Peptid-Synthese, wie sie z. B. in Houben-Weyl, 1.c., Band, Band 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder Dimethylaminopropy-lethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z. B. solche, in denen reaktive Gruppen intermediär durch Schutzgruppen blockiert sind. Die Aminosäurederivate III können z. B. in Form ihrer aktivierten Ester verwendet werden, die zweckmäßig in situ gebildet werden, z. B. durch Zusatz von HOBt oder N-Hydroxysuccinimid.

Die Ausgangsstoffe der Formeln II und III sind größenteils bekannt. Sofern sie nicht bekannt sind, können sie nach bekannten Methoden, z. B. den oben angegebenen Methoden der Peptidsynthese und der Abspaltung von Schutzgruppen, hergestellt werden.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann insbesondere eine Verbindung der Formel I, worin X von H verschieden ist, in eine Verbindung der Formel I (X = H) umgewandelt werden, zweckmäßig durch Hydrogenolyse, falls X = CBZ ist, sonst durch selektive Solvolyse. Falls X = BOC ist, kann man z. B. mit HCl in Dioxan bei Raumtemperatur die BOC-Gruppe abspalten.

Andererseits kann man in einer Verbindung der Formel I gegebenenfalls vorhandene freie Amino- und/oder Hydroxygruppen acylieren, vorzugsweise nach einer der oben für die Umsetzung der Verbindungen der Formeln II und III beschriebenen Methoden. Insbesondere kann eine Verbindung der Formel I, worin k = 0 ist und X-Z- HO- oder H-NR⁹-bedeutet, mit einer Säure der Formel X-OH (worin X von H verschieden ist) oder einem ihrer funktionellen Derivate nach einer dieser Methoden acyliert werden.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z. B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z. B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als InhalationsSpray können Sprays verwendet werden, die den Wirkstoff entweder gelöst oder suspendiert in einem Treibgasgemisch (z. B. Fluorchlor-kohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-163237 beschriebenen Verbindungen verabreicht, vorzugsweise in Dosierungen zwischen etwa 10 mg und 10 g, insbesondere zwischen 50 mg und 5 g pro Dosierungseinheit. Die tägliche Dosierung liegt vorzugsweise zwischen etwa 0,5 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Renin-abhängige Hypertonie und Hyperaldosteronismus können wirksam durch Verabreichung von Dosen zwischen etwa 10 und 300 mg/kg Körpergewicht behandelt werden. Für diagnostische Zwecke können die neuen Aminosäurederivate in Einzeldosen zwischen etwa 0,1 und 10 mg/kg Körpergewicht verabfolgt werden.

Vor- und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation.

### Beispiel 1

Eine Lösung von 4,62 g 1-Amino-2-(H-AHCP-Ile-aminomethyl)-4,5-dimethylpyrazin [erhältlich durch Hydrolyse von BOC-Ile-(N-2-amino-5,6-dimethyl-3-pyrazinylmethylamid) (F. 174°, Zers.) mit 4 n HCl in Dioxan zu H-Ile-(N-2-amino-5,6-dimethyl-3-pyrazinylmethyl-amid), Kondensation mit BOC-AHCP-OH zu BOC-AHCP-Ile-(N-2-amino-5,6-dimethyl-3-pyrazinylmethyl-amid) (F. 175°, Zers.) und Abspaltung der BOC-Gruppe] in 60 ml Dichlormethan wird mit 1,01 g N-Methylmorpholin versetzt. Unter Rühren gibt man 1,75 g BOC-Gly-OH, 1,35 g HOBt und eine Lösung von 2,06 g DCCI in 50 ml Dichlormethan hinzu, rührt 14 Std. bei 2-6°, filtriert den ausgeschiedenen Dicyclohexylharnstoff ab und dampft das Filtrat ein. Man arbeitet wie üblich auf und erhält BOC-Gly-AHCP-Ile-(N-2-amino-5,6-dimethyl-3-pyrazinylmethyl-amid), F. 140° (Zers.).

Analog erhält man durch Abspaltung der BOC-Gruppe das 1-Amino-2-(H-Gly-AHCP-Ile-aminomethyl)-4,5-dimethylpyrazin und daraus mit BOC-PheOH das BOC-Phe-Gly-AHCP-Ile-(N-2-amino-5,6-dimethyl-3-pyrazinylmethyl-amid), F. 164° (Zers.).

Analog erhält man IPOC-Gly-AHCP-Ile-(N-2-amino-5,6-dimethyl-3-pyrazinylmethyl-amid) CBZ-Gly-AHCP-Ile-(N-2-amino-5,6-dimethyl-3-pyrazinylmethyl-amid) POA-Gly-AHCP-Ile-(N-2-amino-5,6-dimethyl-3-pyrazinylmethyl-amid) Acetyl-Gly-AHCP-Ile-(N-2-amino-5,6-dimethyl-3-pyrazinylmethyl-amid) Propionyl-Gly-AHCP-Ile-(N-2-amino-5,6-dimethyl-3-pyrazinylmethyl-amid) Isovaleryl-Gly-AHCP-Ile-(N-2-amino-5,6-dimethyl-3-pyrazinylmethyl-amid) Benzoyl-Gly-AHCP-Ile-(N-2-amino-5,6-dimethyl-3-pyrazinylmethyl-amid) BOC-Gly-AHCP-Ile-NH₂, F. 160-164° BOC-Gly-AHCP-Leu-NH₂ BOC-Gly-AHCP-Phe-NH₂ BOC-Gly-AHCP-Val-NH₂ BOC-Gly-AHCH-Ile-NH₂ BOC-Gly-Sta-Ile-NH₂ BOC-Gly-AHPP-Ile-NH₂ BOC-Gly-AHCP-Leu-(N-2-amino-5,6-dimethyl-3-pyrazinylmethyl-amid) BOC-Gly-AHCP-Phe-(N-2-amino-5,6-dimethyl-3-pyrazinylmethyl-amid) BOC-Gly-AHCP-Val-(N-2-amino-5,6-dimethyl-3-pyrazinylmethyl-amid) BOC-Gly-AHCP-Ile-(N-3-pyridylmethyl-amid) BOC-Gly-AHCP-Ile-(N-4-amino-2-methyl-5-pyrimidyl-methyl-amid),F. 158-160° BOC-Gly-AHCP-Ile-(N-m-aminomethyl-benzyl-amid) BOC-Sar-AHCP-Ile-(N-2-amino-5,6-dimethyl-3-pyrazinylmethyl-amid) BOC-Sar-AHCP-Ile-(N-3-pyridylmethyl-amid) BOC-Sar-AHCP-Ile-(N-4-amino-2-methyl-5-pyrimidinylmethyl-amid),F. 122° (Zers.) BOC-Sar-AHCP-(N-m-aminomethyl-benzyl-amid) IPOC-Phe-Gly-AHCP-Ile-(N-2-amino-5,6-dimethyl-3-pyrazinylmethyl-amid), F. 187 (Zers.) CBZ-Phe-Gly-AHCP-Ile-(N-2-amino-5,6-dimethyl-3-pyrazinylmethyl-amid), F. 137° (Zers.) POA-Phe-Gly-AHCP-Ile-(N-2-amino-5,6-dimethyl-3-pyrazinylmethyl-amid) Acetyl-Phe-Gly-AHCP-Ile-(N-2-amino-5,6-dimethyl-3-pyrazinylmethyl-amid) Propionyl-Phe-Gly-AHCP-Ile-(N-2-amino-5,6-dimethyl-3-pyrazinylmethyl-amid) Isovaleryl-Phe-Gly-AHCP-Ile-(N-2-amino-5,6-dimethyl-3-pyrazinylmethyl-amid), F. 196-198° Benzoyl-Phe-Gly-AHCP-Ile-(N-2-amino-5,6-dimethyl-3-pyrazinylmethyl-amid) BOC-Phe-Gly-AHCP-Ile-NH₂, F. 124-126° BOC-Phe-Gly-AHCP-Leu-NH₂ BOC-Phe-Gly-AHCP-Phe-NH₂ BOC-Phe-Gly-AHCP-Val-NH₂ BOC-Phe-Gly-AHCH-Ile-NH₂ BOC-Phe-Gly-Sta-Ile-NH₂ BOC-Phe-Gly-AHPP-Ile-NH₂ BOC-Phe-Gly-AHCP-(N-2-amino-5,6-dimethyl-3-pyrazinylmethyl-amid) BOC-Phe-Gly-AHCP-Leu-(N-2-amino-5,6-dimethyl-3-pyrazinylmethyl-amid) BOC-Phe-Gly-AHCP-Phe-(N-2-amino-5,6-dimethyl-3-pyrazinylmethyl-amid) BOC-Phe-Gly-AHCP-Val-(N-2-amino-5,6-dimethyl-3-pyrazinylmethyl-amid) BOC-Phe-Gly-AHCP-Ile-(N-3-pyridylmethyl-amid), F. 132-134° BOC-Cal-Gly-AHCP-Ile-(N-3-pyridylmethyl-amid) BOC-*α*-Nal-Gly-AHCP-Ile-(N-3-pyridylmethyl-amid) BOC-*β*-Nal-Gly-AHCP-Ile-(N-3-pyridylmethyl-amid) BOC-L-(2-amino-4-phenylbutyryl)-Gly-AHCP-Ile-(N-3-pyridylmethyl-amid) BOC-L-(2-amino-5-phenylvaleryl)-Gly-AHCP-Ile-(N-3-pyridylmethyl-amid) BOC-L-[2-amino-3-(2-dekalyl)-propionyl]-Gly-AHCP-Ile-(N-3-pyridylmethyl-amid) N-Isobutylcarbamoyl-Phe-Gly-AHCP-Ile-(N-2-amino-5,6-dimethyl-3-pyrazinylmethyl-amid) N-Morpholinocarbonyl-Phe-Gly-AHCP-Ile-(N-2-amino-5,6-dimethyl-3-pyrazinylmethyl-amid), F. 124° (Zers.).

### Beispiel 2

a) Analog Beispiel 1 erhält man aus BOC-Gly-OH und AHCP-OMe das BOC-Gly-AHCP-OMe. (Diese Verbindung wird nicht beansprucht).
   Analog erhält man BOC-Gly-AHCP-Ile-OMe BOC-Gly-AHCP-Leu-OMe BOC-Gly-AHCP-Phe-OMe BOC-Gly-AHCP-Val-OMe BOC-Phe-Gly-AHCP-Ile-OMe, F. 87-90° BOC-Phe-Gly-AHCP-Leu-OMe BOC-Phe-Gly-AHCP-Phe-OMe BOC-Phe-Gly-AHCP-Val-OMe.
b) Ein Gemisch von 1 g BOC-Gly-AHCP-OMe, 50 ml Dioxan und 20 ml 2 n NaOH (wässerig) wird 3 Std. bei 20° gerührt. Nach üblicher Aufarbeitung erhält man BOC-Gly-AHCP-OH, F. 50 °. (Diese Verbindung wird nicht beansprucht).
   Analog erhält man BOC-Gly-AHCP-Ile-OH BOC-Gly-AHCP-Leu-OH BOC-Gly-AHCP-Phe-OH BOC-Gly-AHCP-Val-OH BOC-Phe-Gly-AHCP-Ile-OH, F. 143° (Zers.) BOC-Phe-Gly-AHCP-Leu-OH BOC-Phe-Gly-AHCP-Phe-OH BOC-Phe-Gly-AHCP-Val-OH.

### Beispiel 3

Analog Beispiel 1 erhält man aus (N,N-Dibenzylcarbamoyl)-Gly-OH und H-AHCP-Ile-(N-3-pyridylmethyl-amid) das (N,N-Dibenzylcarbamoyl)-Gly-AHCP-Ile-(N-3-pyridylmethylamid), F. 66° (Zers.)

Analog erhält man IPOC-Gly-AHCP-Ile-(N-3-pyridylmethyl-amid) CBZ-Gly-AHCP-Ile-(N-3-pyridylmethyl-amid) POA-Gly-AHCP-Ile-(N-3-pyridylmethyl-amid) Acetyl-Gly-AHCP-Ile-(N-3-pyridylmethyl-amid) Propionyl-Gly-AHCP-Ile-(N-3-pyridylmethyl-amid) Isovaleryl-Gly-AHCP-Ile-(N-3-pyridylmethyl-amid) Benzoyl-Gly-AHCP-Ile-(N-3-pyridylmethyl-amid) (N-Benzylcarbamoyl)-Gly-AHCP-Ile-(N-3-pyridylmethyl-amid) (N-2-Pyridylmethyl-carbamoyl)-Gly-AHCP-Ile-(N-3-pyridylmethyl-amid) (N-Benzyl-N-butyl-carbamoyl)-Gly-AHCP-Ile-(N-3-pyridylmethyl-amid) (N-Benzyl-N-2-phenylethyl-carbamoyl)-Gly-AHCP-Ile-(N-3-pyridylmethyl-amid) (N,N-Bis-2-phenylethyl-carbamoyl)-Gly-AHCP-Ile-(N-3-pyridylmethyl-amid) (N-2-Morpholinoethyl-N-1-naphthylmethyl-carbamoyl)-Gly-AHCP-Ile-(N-3-pyridylmethyl-amid) (N-Benzyl-carbamoyl)-Gly-AHCP-Ile-(N-4-amino-2-methyl-5-pyrimidinyl-methyl-amid) (N-2-Pyridylmethyl-carbamoyl)-Gly-AHCP-Ile-(N-4-amino-2-methyl-5-pyrimidinyl-methyl-amid) (N-Benzyl-N-butyl-carbamoyl)-Gly-AHCP-Ile-(N-4-amino-2-methyl-5-pyrimidinyl-methyl-amid), F. 109-110° (N,N-Dibenzyl-carbamoyl)-Gly-AHCP-Ile-(N-4-amino-2-methyl-5-pyrimidinyl-methyl-amid) (N-Benzyl-N-2-phenylethyl-carbamoyl)-Gly-AHCP-Ile-(N-4-amino-2-methyl-5-pyrimidinyl-methyl-amid) (N,N-Bis-2-phenylethyl-carbamoyl)-Gly-AHCP-Ile-(N-4-amino-2-methyl-5-pyrimidinyl-methyl-amid), F. 211-213° (N-2-Morpholinoethyl-N-1-naphthylmethyl-carbamoyl)-Gly-AHCP-Ile-(N-4-amino-2-methyl-5-pyrimidinyl-methyl-amid) (N-Benzyl-carbamoyl)-Gly-AHCP-Ile-(N-2-amino-5,6-dimethyl-3-pyrazinyl-methyl-amid), F. 143-144 (N-2-Pyridylmethyl-carbamoyl)-Gly-AHCP-Ile-(N-2-amino-5,6-dimethyl-3-pyrazinyl-methyl-amid), F. 207 (Zers.) (N-Benzyl-N-butyl-carbamoyl)-Gly-AHCP-Ile-(N-2-amino-5,6-dimethyl-3-pyrazinyl-methyl-amid), F. 100° (Zers.) (N,N-Dibenzyl-carbamoyl)-Gly-AHCP-Ile-(N-2-amino-5,6-dimethyl-3-pyrazinyl-methyl-amid), F. 101-104° (N-Benzyl-N-2-phenylethyl-carbamoyl)-Gly-AHCP-Ile-(N-2-amino-5,6-dimethyl-3-pyrazinyl-methyl-amid), F. 89-91 ° (N,N-Bis-2-phenylethyl-carbamoyl)-Gly-AHCP-Ile-(N-2-amino-5,6-dimethyl-3-pyrazinyl-methyl-amid) (N-2-Morpholinoethyl-N-1-naphthylmethyl-carbamoyl)-Gly-AHCP-Ile-(N-2-amino-5,6-dimethyl-3-pyrazinyl-methyl-amid) BOC-Gly-AHCH-Ile-(N-3-pyridylmethyl-amid) BOC-Gly-Sta-Ile-(N-3-pyridylmethyl-amid) BOC-Gly-AHPP-Ile-(N-3-pyridylmethyl-amid) N-[3S-Hydroxy-4S-(BOC-Gly-amino)-octanoyl]-Ile-(N-3-pyridylmethyl-amid) BOC-Gly-AHCH-Ile-(N-2-amino-5,6-dimethyl-3-pyrazinylmethyl-amid) BOC-Gly-Sta-Ile-(N-2-amino-5,6-dimethyl-3-pyrazinylmethyl-amid) BOC-Gly-AHPP-Ile-(N-2-amino-5,6-dimethyl-3-pyrazinylmethyl-amid) N-[3S-hydroxy-4S-(BOC-Gly-amino)-octanoyl]-Ile-(N-2-amino-5,6-dimethyl-3-pyrazinylmethyl-amid) BOC-Phe-Gly-AHCP-Ile-(N-3-pyridylmethyl-amid), F. 132-134° IPOC-Phe-Gly-AHCP-Ile-(N-3-pyridylmethyl-amid) CBZ-Phe-Gly-AHCP-Ile-(N-3-pyridylmethyl-amid) POA-Phe-Gly-AHCP-Ile-(N-3-pyridylmethyl-amid) Acetyl-Phe-Gly-AHCP-Ile-(N-3-pyridylmethyl-amid) Propionyl-Phe-Gly-AHCP-Ile-(N-3-pyridylmethyl-amid) Isovaleryl-Phe-Gly-AHCP-Ile-(N-3-pyridylmethyl-amid) Benzoyl-Phe-Gly-AHCP-Ile-(N-3-pyridylmethyl-amid) N-[3-(BOC-Phe)-propionyl]-AHCP-Ile-(N-isobutylamid) N-[3-(BOC-Phe)-propionyl]-AHCP-Ile-(N-isopentylamid) N-[3-(BOC-Phe)-propionyl]-AHCP-Ile-(N-3-pyridylmethyl-amid) N-[3-(Benzoyl-Phe)-propionyl]-AHCP-Ile-(N-2-amino-5,6-dimethyl-3-pyrazinylmethyl-amid), F. 182-184° N-[4-(BOC-Phe)-3-thiabutyryl]-AHCP-Ile-(N-3-pyridylmethyl-amid) [F. 118-120°; mit 4-(BOC-Phe)-3-thiabuttersäure, F. 110-111°, erhältlich aus BOC-Phe-CH₂Br und HS-CH₂COOH in THF in Gegenwart von NaOCH₃] N-[4-(BOC-Phe)-3-thiabutyryl]-AHCP-Ile-(N-isobutylamid) N-[4-(BOC-Phe)-3-thiabutyryl]-AHCP-Ile-(N-isopentylamid) N-[4-(BOC-Phe)-3-thiabutyryl]-AHCP-Ile-(N-2-amino-5,6-dimethyl-3-pyrazinylmethyl-amid), F. 153-155°. N-[4-(Morpholinocarbonyl-Phe)-3-thiabutyryl]-AHCP-(N-isopentylamid) BOC-Phe-Gly-AHCH-Ile-(N-3-pyridylmethyl-amid) BOC-Phe-Gly-Sta-Ile-(N-3-pyridylmethyl-amid) BOC-Phe-Gly-AHPP-Ile-(N-3-pyridylmethyl-amid) N-[3S-Hydroxy-4S-(BOC-Phe-Gly-amino)-octanoyl]-Ile-(N-3-pyridylmethyl-amid) BOC-Phe-Gly-AHCH-Ile-(N-2-amino-5,6-dimethyl-3-pyrazinylmethyl-amid) BOC-Phe-Gly-Sta-Ile-(N-2-amino-5,6-dimethyl-3-pyrazinylmethyl-amid) BOC-Phe-Gly-AHPP-Ile-(N-2-amino-5,6-dimethyl-3-pyrazinylmethyl-amid) N-[3S-hydroxy-4S-(BOC-Phe-Gly-amino)-octanoyl]-Ile-(N-2-amino-5,6-dimethyl-3-pyrazinylmethyl-amid), F. 183-185° N-[3-(BOC-Phe)-propionyl]-AHCP-Ile-(N-2-amino-5,6-dimethyl-3-pyrazinylmethyl-amid), F. 167-169°.

Mit H-AHCP-Ile-NH₂ erhält man analog: IPOC-Gly-AHCP-Ile-NH₂ BOC-Gly-AHCP-Ile-NH₂ CBZ-Gly-AHCP-Ile-NH₂ POA-Gly-AHCP-Ile-NH₂ Acetyl-Gly-AHCP-Ile-NH2 Propionyl-Gly-AHCP-Ile-NH₂ Isovaleryl-Gly-AHCP-Ile-NH₂ Benzoyl-Gly-AHCP-Ile-NH₂ IPOC-Phe-Gly-AHCP-Ile-NH₂ BOC-Phe-Gly-AHCP-Ile-NH₂, F. 124-126° CBZ-Phe-Gly-AHCP-Ile-NH₂ POA-Phe-Gly-AHCP-Ile-NH₂ Acetyl-Phe-Gly-AHCP-Ile-NH₂ Propionyl-Phe-Gly-AHCP-Ile-NH₂ Isovaleryl-Phe-Gly-AHCP-Ile-NH₂ Benzoyl-Phe-Gly-AHCP-Ile-NH₂ N-[3-(BOC-Phe)-propionyl]-AHCP-Ile-NH₂ N-[4-(BOC-Phe)-3-thiabutyryl]-AHCP-Ile-NH₂, F. 125-128°.

### Beispiel 4

Analog Beispiel 1 erhält man aus 3-(N-Benzyl-N-butylcarbamoyl)-propionsäure (erhältlich aus Succinanhydrid und N-Benzyl-N-butylamin) und H-AHCP-Ile-(N-4-amino-2-methyl-5-pyrimidinyl-methyl-amid) das [3-(N-Benzyl-N-butyl-carbamoyl)-propionyl-AHCP]-Ile-(N-4-amino-2-methyl-5-pyrimidinyl-methyl-amid), F. 88° Analog erhält man [3-(N,N-Dibenzyl-carbamoyl)-propionyl]-AHCP-Ile-(N-4-amino-2-methyl-5-pyrimidinyl-methyl-amid) [3-(N-Benzyl-N-pentyl-carbamoyl)-propionyl]-AHCP-Ile-(N-4-amino-2-methyl-5-pyrimidinyl-methyl-amid) [3-N-Benzyl-N-2-phenylethyl-carbamoyl)-propionyl]-AHCP-Ile-(N-4-amino-2-methyl-5-pyrimidinyl-methyl-amid) [3-(N-2-Morpholinoethyl-N-1-naphthylmethyl-carbamoyl)-propionyl]-AHCP-Ile-(N-4-amino-2-methyl-5-pyrimidinyl-methyl-amid) [3-(N-Benzyl-N-butyl-carbamoyl)-propionyl]-AHCP-Ile-(N-3-pyridyl-methyl-amid) [3-(N-Benzyl-N-pentyl-carbamoyl)-propionyl]-AHCP-Ile-(N-3-pyridyl-methyl-amid) [3-(N,N-Dibenzyl-carbamoyl)-propionyl]-AHCP-Ile-(N-3-pyridyl-methyl-amid) [3-(N-Benzyl-N-2-phenylethyl-carbamoyl)-propionyl]-AHCP-Ile-(N-3-pyridyl-methyl-amid) [3-(N-2-Morpholinoethyl-N-1-naphthylmethyl-carbamoyl)-propionyl]-AHCP-Ile-(N-3-pyridyl-methyl-amid) [3-(N-Benzyl-N-butyl-carbamoyl)-propionyl]-AHCP-Ile-N-(2-amino-5,6-dimethyl-3-pyrazinyl-methyl-amid) [3-(N-Benzyl-N-pentyl-carbamoyl)-propionyl]-AHCP-Ile-N-(2-amino-5,6-dimethyl-3-pyrazinyl-methyl-amid) [3-(N,N-Dibenzyl-carbamoyl)-propionyl]-AHCP-Ile-N-(2-amino-5,6-dimethyl-3-pyrazinyl-methyl-amid) [3-(N-Benzyl-N-2-phenylethyl-carbamoyl)-propionyl]-AHCP-Ile-N-(2-amino-5,6-dimethyl-3-pyrazinyl-methyl-amid) [3-(N-2-Morpholinoethyl-N-1-naphthylmethyl-carbamoyl)-propionyl]-AHCP-Ile-N-(2-amino-5,6-dimethyl-3-pyrazinylmethyl-amid).

### Beispiel 5

Analog Beispiel 1 erhält man aus (N,N-Diben-zyl-carbamoyl)-Gly-AHCP-OH und Isoleucin-(N-2-amino-5,6-dimethyl-3-pyrazinylmethyl-amid) das (N,N-Dibenzyl-carbamoyl)-Gly-AHCP-Ile-(N-2-amino-5,6-dimethyl-3-pyrazinylmethyl-amid), F. 101-104°.

Analog erhält man mit den entsprechenden Aminoverbindungen: BOC-Phe-Gly-AHCP-Ile-(N-3-pyridylmethyl-amid), F. 132-134°.

### Beispiel 6

Analog Beispiel 1 erhält man aus BOC-Gly-AHCP-Ile-OH und H-Phe-NH₂ das BOC-Gly-AHCP-Ile-Phe-NH₂.

Analog erhält man mit den entsprechenden Aminosäureamiden: BOC-Gly-AHCP-Ile-Ile-NH₂ BOC-Gly-AHCP-Ile-Leu-NH₂ BOC-Gly-AHCP-Ile-Val-NH₂ BOC-Phe-Gly-AHCP-Ile-Ile-NH₂ BOC-Phe-Gly-AHCP-Ile-Leu-NH₂ BOC-Phe-Gly-AHCP-Ile-Phe-NH₂, F. 188° (Zers.) BOC-Phe-Gly-AHCP-Ile-Val-NH₂.

### Beispiel 7

Analog Beispiel 1 erhält man aus BOC-Gly-AHCP-Ile-OH und 2-Amino-3-aminomethyl-5,6-di-methylpyrazin das BOC-Gly-AHCP-Ile-(N-2-amino-5,6-dimethyl-3-pyrazinylmethyl-amid), F. 140° (Zers.).

Analog erhält man BOC-Gly-AHCP-Ile-(N-2-pyridyl-methyl-amid) BOC-Gly-AHCP-Ile-(N-3-pyridyl-methyl-amid) BOC-Gly-AHCP-Ile-(N-4-pyridyl-methyl-amid) BOC-Gly-AHCP-Ile-(N-2-amino-5,6-dimethyl-3-pyridyl-methyl-amid) BOC-Gly-AHCP-Ile-(N-2-hydroxy-4,6-dimethyl-3-pyridyl-methyl-amid) BOC-Gly-AHCP-Ile-(N-m-aminomethylbenzyl-amid) BOC-Gly-AHCP-Ile-[N-2-(2-methyl-3-hydroxy-4-hydroxymethyl-5-pyridyl)-ethyl-amid] BOC-Gly-AHCP-Ile-(N-5-pyrimidinyl-methyl-amid) BOC-Gly-AHCP-Ile-(N-4-amino-2-methyl-5-pyrimidinyl-methyl-amid) BOC-Gly-AHCP-Ile-(N-2-hydroxy-4-amino-6-methyl-3-pyridyl-methyl-amid) BOC-Gly-AHCP-Ile-[N-2-(4-methyl-5-thiazolyl)-ethyl-amid] BOC-Gly-AHCP-Ile-(N-5-tetrazolyl-methyl-amid), F. 124° BOC-Gly-AHCP-Ile-(N-2-morpholinoethyl-amid) BOC-Gly-AHCP-Ile-[N-2-(4-pyridyl)-ethyl-amid] BOC-Gly-AHCP-Leu-(N-m-aminomethylbenzyl-amid). (N-Benzyl-carbamoyl)-Gly-AHCP-Ile-(N-m-aminomethyl-benzyl-amid) (N-2-Pyridylmethyl-carbamoyl)-Gly-AHCP-Ile-(N-m-aminomethyl-benzyl-amid) (N-Benzyl-N-butyl-carbamoyl)-Gly-AHCP-Ile-(N-m-aminomethyl-benzyl-amid), F. 97-99° (N,N-Dibenzyl-carbamoyl)-Gly-AHCP-Ile-(N-m-aminomethyl-benzyl-amid) (N-Benzyl-N-2-phenylethyl-carbamoyl)-Gly-AHCP-Ile-(N-m-aminomethyl-benzyl-amid) (N,N-Bis-2-phenylethyl-carbamoyl)-Gly-AHCP-Ile-(N-m-aminomethyl-benzyl-amid) (N-2-Morpholinoethyl-N-1-naphthylmethyl-carbamoyl)-Gly-AHCP-Ile-(N-m-aminomethyl-benzyl-amid) [3-(N-Benzyl-N-butyl-carbamoyl)-propionyl]-AHCP-Ile-N-(m-aminomethyl-benzyl-amid) [3-(N-Benzyl-N-pentyl-carbamoyl)-propionyl]-AHCP-Ile-N-(m-aminomethyl-benzyl-amid) [3-(N,N-Dibenzyl-carbamoyl)-propionyl]-AHCP-Ile-N-(m-aminomethyl-benzyl-amid) [3-(N-Benzyl-N-2-phenylethyl-carbamoyl)-propionyl]-AHCP-Ile-N-(m-aminomethyl-benzyl-amid) [3-(N-2-Morpholinoethyl-N-1-naphthylmethyl-carbamoyl)-propionyl]-AHCP-Ile-N-(m-aminomethyl-benzyl-amid).

Analog erhält man die folgenden Gly-AHCP-Ile-(N-m-aminomethyl-benzyl-amide): Hexanoyl-Heptanoyl-(2-Propyl-valeryl)-(3-Phenyl-propionyl)-(4-Phenyl-butyryl)-(2-Benzyl-butyryl)-(2-Benzyl-hexanoyl)-(2-Benzyl-heptanoyl)-, F. 100-105° (2-Benzyl-4-phenyl-butyryl)-POA-[2-Benzyl-4-(2-oxopyrrolidino)-butyryl]-[2-Benzyl-4-(4-pyridyl)-butyryl]-(2-Benzyl-3-ethoxycarbonyl-propionyl)-[2-(1-Naphthylmethyl)-3-ethoxycarbonyl-propionyl]-[2-(1-Naphthylmethyl)-3-(N-isopropyl-carbamoyl)-propionyl]-[2-(1-Naphthylmethyl)-3-(N-2-phenylethyl-carbamoyl)-propionyl]-[2-(1-Naphthylmethyl)-3-morpholinocarbonyl-propionyl]-(2-Benzyl-4-oxo-hexanoyl) - (2-Benzyl-4-oxo-5,5-dimethyl-hexanoyl)-, F. 102-105° (2-Benzyl-4-oxo-6-methyl-heptanoyl)-(2-Benzyl-4-oxo-4-phenyl-butyryl)-[2-Benzyl-4-oxo-4-(2-pyridyl-butyryl]-BOC-Phe-Gly-AHCP-Ile-(N-2-amino-5,6-dimethyl-3-pyrazinylmethyl-amid), F. 164° (Zers.). BOC-Phe-Gly-AHCP-Ile-(N-2-pyridylmethyl-amid) BOC-Phe-Gly-AHCP-Ile-(N-3-pyridylmethyl-amid), F. 132-134° BOC-Phe-Gly-AHCP-Ile-(N-4-pyridylmethyl-amid) BOC-Phe-Gly-AHCP-Ile-(N-m-aminomethylbenzyl-amid), F. 146-149° BOC-Phe-Gly-AHCP-Ile-[N-2-(2-methyl-3-hydroxy-4-hydroxymethyl-5-pyridyl)-ethyl-amid] BOC-Phe-Gly-AHCP-Ile-(N-2-methyl-4-amino-5-pyrimidylmethyl-amid), F. 129-131° BOC-Phe-Gly-AHCP-Ile-(N-2-hydroxy-4-amino-6-methyl-3-pyridyl-methyl-amid), F. 173-175° BOC-Phe-Gly-AHCP-Ile-[N-2-(4-methyl-5-thiazolyl)-ethylamid] BOC-Phe-Gly-AHCP-Ile-(N-5-tetrazolyl-methyl-amid), F. 155° (Zers.) BOC-Phe-Gly-AHCP-Ile-(N-2-morpholinoethyl-amid) BOC-Phe-Gly-AHCP-Ile-[N-2-(4-pyridyl)-ethyl-amid], F. 106-108° BOC-Phe-Gly-AHCP-Leu-(N-m-aminomethylbenzyl-amid).

### Beispiel 8

Eine Lösung von 1 g BOC-Gly-AHCP-Leu-NH₂ in 20 ml 4 n HCl in Dioxan wird 30 Min. bei 20° gerührt und dann eingedampft. Man erhält H-Gly-AHCP-Leu-NH₂ in Form des hygroskopischen Hydrochlorids.

Analog erhält man aus den entsprechenden BOC-Derivaten: H-Gly-AHCP-Ile-(N-4-amino-2-methyl-5-pyrimidinyl-methy-amid) H-Gly-AHCP-Ile-(N-3-pyridylmethyl-amid) H-Gly-AHCP-Ile-(N-2-amino-5,6-dimethyl-3-pyrazinylmethyl-amid) H-Gly-AHCP-Ile-(N-m-aminomethyl-benzyl-amid) H-Sar-AHCP-Ile-(N-4-amino-2-methyl-5-pyrimidinyl-methyl-amid) H-Sar-AHCP-Ile-(N-3-pyridylmethyl-amid) H-Sar-AHCP-Ile-(N-2-amino-5,6-dimethyl-3-pyrazinylmethyl-amid) H-Sar-AHCP-Ile-(N-m-aminomethyl-benzyl-amid) H-Phe-Gly-AHCP-Ile-NH₂ H-Phe-Gly-AHCP-Ile-(N-3-pyridylmethyl-amid) H-Phe-Gly-AHCP-Ile-(N-2-amino-5,6-dimethyl-3-pyrazinylmethyl-amid).

### Beispiel 9

Man löst 1 g CBZ-Gly-AHCP-Ile-NH₂ in 10 ml Ethanol, hydriert an 0,5 g 10 %igem Pd-C bei 20° und 1 bar bis zum Stillstand, filtriert, dampft ein und erhält nach chromatographischer Reinigung H-Gly-AHCP-Ile-NH₂.

Analog erhält man H-Phe-Gly-AHCP-Ile-NH₂.

### Beispiel 10

Analog Beispiel 9 erhält man aus N-[35-CBZ-amino-4S-(BOC-Gly-amino)-5-cyclohexyl-pentanoyl]-Ile-NH₂ das BOC-Gly-DACP-Ile-NH₂.

Analog erhält man durch Hydrogenolyse der entsprechenden CBZ-Derivate BOC-Gly-DACH-Ile-NH₂ BOC-Gly-DAMH-Ile-NH₂ BOC-Gly-DAPP-Ile-NH₂ BOC-Gly-DACP-Ile-(N-3-pyridylmethyl-amid) BOC-Gly-DACH-Ile-(N-3-pyridylmethyl-amid) BOC-Gly-DAMH-Ile-(N-3-pyridylmethyl-amid) BOC-Gly-DAPP-Ile-(N-3-pyridylmethyl-amid) (2-Benzyl-heptanoyl)-Gly-DACP-Ile-(N-2-amino-5,6-dimethyl-3-pyrazinyl-methyl-amid) (2-Benzyl-heptanoyl)-Gly-DACH-Ile-(N-2-amino-5,6-dimethyl-3-pyrazinyl-methyl-amid) (2-Benzyl-heptanoyl)-Gly-DAMH-Ile-(N-2-amino-5,6-dimethyl-3-pyrazinyl-methyl-amid) (2-Benzyl-heptanoyl)-Gly-DAPP-Ile-(N-2-amino-5,6-dimethyl-3-pyrazinyl-methyl-amid) BOC-Phe-Gly-DACP-Ile-NH₂ BOC-Phe-Gly-DACH-Ile-NH₂ BOC-Phe-Gly-DAMH-Ile-NH₂ BOC-Phe-Gly-DAPP-Ile-NH₂ BOC-Phe-Gly-DACP-Ile(N-3-pyridylmethyl-amid), F. 103-105° BOC-Phe-Gly-DACH-Ile(N-3-pyridylmethyl-amid) BOC-Phe-Gly-DAMH-Ile(N-3-pyridylmethyl-amid) BOC-Phe-Gly-DAPP-Ile(N-3-pyridylmethyl-amid) BOC-Phe-Gly-DACP-Ile-(N-2-amino-5,6-dimethyl-3-pyrazinylmethyl-amid), F. 137° BOC-Phe-Gly-DACH-Ile-(N-2-amino-5,6-dimethyl-3-pyrazinylmethyl-amid) BOC-Phe-Gly-DAMH-Ile-(N-2-amino-5,6-dimethyl-3-pyrazinylmethyl-amid) BOC-Phe-Gly-DAPP-Ile-(N-2-amino-5,6-dimethyl-3-pyrazinylmethyl-amid).

### Beispiel 11

Analog Beispiel 1 erhält man aus 2-Benzylheptansäure und H-Gly-AHCP-Ile-(N-4-amino-2-methyl-5-pyrimidinyl-methyl-amid) das (2-Benzyl-heptanoyl)-Gly-AHCP-Ile-(N-4-amino-2-methyl-5-pyrimidinyl-methyl-amid), F. 110°. Analog erhält man die folgenden Gly-AHCP-Ile-(N-4-amino-2-methyl-5-pyrimidinyl-methyl-amide): Hexanoyl-Heptanoyl-(2-Propylvaleryl)-(3-Phenyl-propionyl)-, F. 103° (4-Phenyl-butyryl)-(2-Benzyl-butyryl)-, F. 116-118° (2-Benzyl-hexanoyl)-, F. 103° (2-Benzyl-5-methyl-hexanoyl)-, F. 110-113° (2-Benzyl-heptanoyl)-(2-Benzyl-4-phenyl-butyryl)-, F. 103-106° POA-, F. 143-144° [2-Benzyl-4-(2-oxopyrrolidino)-butyryl]-[2-Benzyl-4-(4-pyridyl)-butyryl]-(2-Benzyl-3-ethoxycarbonyl-propionyl)-[2-(1-Naphthylmethyl)-3-ethoxycarbonyl-propionyl]-[2-(1-Naphthylmethyl)-3-(N-isopropyl-carbamoyl)-propionyl]-(-)-[2-(1-Naphthylmethyl)-3-(N-isopropyl-carbamoyl)-propionyl]-, F. 143° (+)-[2-(1-Naphthylmethyl)-3-(N-isopropyl-carbamoyl)-propionyl]-, F. 152° [2-(1-Naphthylmethyl)-3-(N-2-phenylethyl-carbamoyl)-propionyl]-(-)-[2-(1-Naphthylmethyl)-3-(N-2-phenylethyl-carbamoyl)-propionyl]-, F. 137° (+)-[2-(1-Naphthylmethyl)-3-(N-2-phenylethyl-carbamoyl)-propionyl]-,F. 134° [2-(1-Naphthylmethyl)-3-morpholinocarbonyl-propionyl]-(2-Benzyl-4-oxo-hexanoyl)-(2-Benzyl-4-oxo-5,5-dimethyl-hexanoyl)-(-)-(2-Benzyl-4-oxo-5,5-dimethyl-hexanoyl)-, F. 125-128° (+)-(2-Benzyl-4-oxo-5,5-dimethyl-hexanoyl)-, F. 132-135° (2-Benzyl-4-oxo-6-methyl-heptanoyl)-(2-Benzyl-4-oxo-4-phenyl-butyryl)-[2-Benzyl-4-oxo-4-(2-pyridyl)-butyryl]-.

Analog erhält man die folgenden Gly-AHCP-Ile-(N-3-pyridyl-methyl-amide): Hexanoyl-Heptanoyl-(2-Propyl-valeryl)-(3-Phenyl-propionyl)-(4-Phenyl-butyryl)-(2-Benzyl-butyryl)-(2-Benzyl-hexanoyl)-(2-Benzyl-heptanoyl)-(2-Benzyl-4-phenyl-butyryl)-POA-[2-Benzyl-4-(2-oxopyrrolidino)-butyryl]-[2-Benzyl-4-(4-pyridyl)-butyryl]-(2-Benzyl-3-ethoxycarbonyl-propionyl)-[2-(1-Naphthylmethyl)-3-ethoxycarbonyl-propionyl]-[2-(1-Naphthylmethyl)-3-(N-isopropyl-carbamoyl)-propionyl]-[2-(1-Naphthylmethyl)-3-(N-2-phenylethyl-carbamoyl)-propionyl]-[2-(1-Naphthylmethyl)-3-morpholinocarbonyl)-propionyl]-(2-Benzyl-4-oxo-hexanoyl)-(2-Benzyl-4-oxo-5,5-dimethyl-hexanoyl)-(2-Benzyl-4-oxo-6-methyl-heptanoyl)-(2-Benzyl-4-oxo-4-phenyl-butyryl)-[2-Benzyl-4-oxo-4-(2-pyridyl)-butyryl]-.

Analog erhält man die folgenden Gly-AHCP-Ile-(N-2-amino-5,6-dimethyl-3-pyrazinyl-methyl-amide): Hexanoyl-Heptanoyl-(2-Propyl-valeryl)-(3-Phenyl-propionyl)-(4-Phenyl-butyryl)-(2-Benzyl-butyryl)-(2-Benzyl-hexanoyl)-(2-Benzyl-heptanoyl)-, F. 194° (Zers.) (2-Benzyl-4-phenyl-butyryl)-POA-[2-Benzyl-4-(2-oxopyrrolidino)-butyryl]-, F. 120° (Zers.)-[2-Benzyl-4-(4-pyridyl)-butyryl]-(2-Benzyl-3-ethoxycarbonyl-propionyl)-, F. 170°-[2-(1-Naphthylmethyl)-3-ethoxycarbonyl-propionyl]-, F. 161° (Zers.) [2-(1-Naphthylmethyl)-3-(N-isopropyl-carbamoyl)-propionyl]-[2-(1-Naphthylmethyl)-3-(N-2-phenylethyl-carbamoyl)-propionyl]-[2-(1-Naphthylmethyl)-3-morpholinocarbonyl-propionyl]-(2-Benzyl-4-oxo-hexanoyl)-(2-Benzyl-4-oxo-5,5-dimethyl-hexanoyl)-(2-Benzyl-4-oxo-6-methyl-heptanoyl)-(2-Benzyl-4-oxo-4-phenyl-butyryl)-[2-Benzyl-4-oxo-4-(2-pyridyl)-butyryl]-.

Analog erhält man die folgenden Sar-AHCP-Ile-(N-4-amino-2-methyl-5-pyrimidinyl-methyl-amide): (2-Propyl-valeryl)-, F. 86-88°-(2-Benzyl-butyryl)-(2-Benzyl-hexanoyl)-(2-Benzyl-heptanoyl)-, F. 85-88°-(2-Benzyl-4-phenyl-butyryl)-POA-(2-Benzyl-4-oxo-5,5-dimethyl-hexanoyl)-.

Analog erhält man die folgenden Sar-AHCP-Ile-(N-3-pyridyl-methyl-amide): (2-Propyl-valeryl)-(2-Benzyl-butyryl)-(2-Benzyl-hexanoyl)-(2-Benzyl-heptanoyl)-(2-Benzyl-4-phenyl-butyryl)-POA-(2-Benzyl-4-oxo-5,5-dimethyl-hexanoyl)-.

Analog erhält man die entsprechenden Sar-AHCP-Ile-(N-2-amino-5,6-dimethyl-3-pyrazinyl-methyl-amide): (2-Propyl-valeryl)-(2-Benzyl-butyryl)-(2-Benzyl-hexanoyl)-(2-Benzyl-heptanoyl)-(2-Benzyl-4-phenyl-butyryl)-POA-(2-Benzyl-4-oxo-5,5-dimethyl-hexanoyl)-.

Analog erhält man: [2-Benzyl-4-oxo-4-(2-pyridyl)-butyryl]-Gly-AHCP-Leu-NH₂,2 Isomere, F. 116° und F. 128° 2S-Isopropyl-4S-hydroxy-5S-(2-benzyl-heptanoyl-Gly-amino)-6-cyclohexyl-hexanoyl-Ile-NH₂ 2S-Isopropyl-4S-hydroxy-5S-(2-benzyl-heptanoyl-Sar-amino)-6-cyclohexyl-hexanoyl-Ile-NH₂ 2S-Isopropyl-4S-hydroxy-5S-(2-benzyl-heptanoyl-Gly-amino)-6-cyclohexyl-hexanoyl-Ile-(N-4-amino-2-methyl-5-pyrimidinyl-methyl-amid) 2S-Isopropyl-4S-hydroxy-5S-(2-benzyl-heptanoyl-Sar-amino)-6-cyclohexyl-hexanoyl-Ile-(N-4-amino-2-methyl-5-pyrimidinyl-methyl-amid) 2S-Isopropyl-4S-hydroxy-5S-(2-benzyl-4-oxo-5,5-dimethylhexanoyl-Gly-amino)-6-cyclohexyl-hexanoyl-Leu-NH₂ 2S-Isopropyl-4S-hydroxy-5S-(2-benzyl-4-oxo-5,5-dimethylhexanoyl-Sar-amino)-6-cyclohexyl-hexanoyl-Leu-NH₂ 2S-Isopropyl-4S-hydroxy-5S-(2-benzyl-4-oxo-5,5-dimethylhexanoyl-Gly-amino)-6-cyclohexyl-hexanoyl-Ile-(N-4-amino-2-methyl-5-pyrimidyl-methyl-amid) 2S-Isopropyl-4S-hydroxy-5S-(2-benzyl-4-oxo-5,5-dimethylhexanoyl-Sar-amino)-6-cyclohexyl-hexanoyl-Ile-(N-4-amino-2-methyl-5-pyrimidyl-methyl-amid).

### Beispiel 12

Analog Beispiel 4 erhält man mit CBZ-Gly-OH das CBZ-Gly-AHCP-Ile-(N-4-amino-2-methyl-5-pyrimidinyl-methyl-amid), F. 108°.

Analog erhält man die folgenden AHCP-Ile-(N-4-amino-2-methyl-5-pyrimidinyl-methyl-amide): Butyryl-Phe-Gly-(2-Benzyl-octanoyl)-Gly, F. 105-108° (2-Benzyl-6-methyl-heptanoyl)-Gly, F. 158-160° [2-Benzyl-4-(2-pyridyl)-butyryl]-Gly-(2-Benzyl-3-N,N-dimethylcarbamoyl-propionyl)-Gly-(-)-[2-(1-Naphthylmethyl)-3-(N-tert.-butyl-carbamoyl)-propionyl]-Gly-, F. 146° (+)-[2-(1-Naphthylmethyl)-3-(N-tert.-butyl-carbamoyl)-propionyl]-Gly-, F. 173° (-)-[2-(1-Naphthylmethyl)-3-(N-2-(2-pyridyl)-ethylcarbamoyl)-propionyl]-Gly-, F. 126° (+)-[2-(1-Naphthylmethyl)-3-(N-2-(2-pyridyl)-ethylcarbamoyl)-propionyl]-Gly-, F. 146° Ethoxycarbonyl-Phe-Gly-Isopropoxycarbonyl-Phe-Gly-, F. 194° Isopropoxycarbonyl-Phe-Sar-, F. 124-127° 2-(2-Pyridyl)-ethoxycarbonyl-Phe-Gly-BOC-(L-2-aminovaleryl)-Gly-BOC-Cal-Gly-, F. 138-140° BOC-(L-p-jodphenylalanyl)-Gly-, F. 183° (Zers.) BOC-*α*Nal-Gly-, F. 127° BOC-*β*Nal-Gly-, F. 176° (Zers.) BOC-Nle-Gly-, F. 129-131° BOC-(L-3-dekahydro-*α*Nal)-Gly-, F. 155° (Zers.) BOC-(L-homo-phenylalanyl)-Gly-, F. 122° (Zers.) BOC-Trp-Gly-, F. 155° (Zers.) (2-Propylamino-3-phenyl-propionyl)-Gly-(2-Butylamino-3-phenyl-propionyl)-Gly-(2-tert.-Butylamino-3-phenyl-propionyl)-Gly-, F. 130-131° (2-Morpholino-3-phenyl-propionyl)-Gly-, F. 145° (N-Isopropyl-carbamoyl)-Phe-Gly-(N-Isopentyl-carbamoyl)-Phe-Gly-(N,N-Dibutyl-carbamoyl)-Gly-, F. 112° (N-Benzyl-N-ethyl-carbamoyl)-Gly-, F. 123-126° (N-Benzyl-N-pentyl-carbamoyl)-Gly-, F. 110-113° (N-Benzyl-N-isopentyl-carbamoyl)-Gly-, F. 116-119° (N-Benzyl-N-4-methylpentyl-carbamoyl)-Gly-, F. 112-113° (N-Benzyl-N-3,3-dimethylbutyl-carbamoyl)-Gly-, F. 114-118° [N-Benzyl-N-3-(2-oxopyrrolidino)-propyl-carbamoyl]-Gly-[N-Benzyl-N-(3-morpholinopropyl)-carbamoyl]-Gly-, F. 109-110° Pyrrolidinocarbonyl-Phe-Gly-Piperidinocarbonyl-Phe-Gly-Morpholinocarbonyl-Phe-Gly-, F. 106-108° Morpholinocarbonyl-Phe-Sar-, F. 131-133° Piperidinoacetyl-Phe-Gly-Morpholinoacetyl-Nle-Gly-Morpholinoacetyl-Phe-Gly-, F. 157° Thiomorpholinoacetyl-Phe-Gly-S-Oxido-thiomorpholinoacetyl-Phe-Gly-S,S-Dioxido-thiomorpholinoacetyl-Phe-Gly-(3-Morpholino-propionyl)-Phe-Gly-(2-Methoxycarbonyl-pyrrolidino)-carbonyl-Phe-Gly-(2-tert.-Butoxycarbonyl-pyrrolidino)-carbonyl-Phe-Gly-3-(BOC-Phe-amino)-propionyl-3-(Isopropoxycarbonyl-Phe-amino)-propionyl_ 3-(Morpholinoacetyl-Phe-amino)-propionyl-3-(2-Benzyl-heptanoyl-amino)-propionyl-3-[2-(2-Pyridyl)-ethoxycarbonyl-Phe-amino)-propionyl-4-(BOC-Phe-amino)-butyryl-4-(Isopropoxycarbonyl-Phe-amino)-butyryl-4-(Morpholinocarbonyl-Phe-amino)-butyryl-2-Benzyl-4-oxo-heptanoyl-.

### Beispiel 13

Analog Beispiel 4 erhält man mit CBZ-Gly-OH das CBZ-Gly-AHCP-Ile-(N-2-amino-5,6-dimethyl-3-pyrazinyl-methyl-amid). Analog erhält man die folgenden AHCP-Ile-(N-2-amino-5,6-dimethyl-3-pyrazinyl-methyl-amide): Butyryl-Phe-Gly-, F. 188° (2-Benzyl-octanoyl)-Gly-(2-Benzyl-6-methyl-heptanoyl)-Gly-[2-Benzyl-4-(2-pyridyl)-butyryl]-Gly-, Öl (2-Benzyl-3-N,N-dimethylcarbamoyl-propionyl)-Gly-(-)-[2-(1-Naphthylmethyl)-3-(N-tert.-butyl-carbamoyl)-propionyl]-Gly-(+)-[2-(1-Naphthylmethyl)-3-(N-tert.-butyl-carbamoyl)-propionyl]-Gly-(-)-[2-(1-Naphthylmethyl)-3-(N-2-(2-pyridyl)-ethylcarbamoyl)-propionyl]-Gly-(+)-[2-(1-Naphthylmethyl)-3-(N-2-(2-pyridyl)-ethylcarbamoyl)-propionyl]-Gly-Ethoxycarbonyl-Phe-Gly-, F. 172-173° Isopropoxycarbonyl-Phe-Gly-Isopropoxycarbonyl-Phe-Sar-2-(2-Pyridyl)-ethoxycarbonyl-Phe-Gly-, F. 171° BOC-(L-2-aminovaleryl)-Gly-BOC-Cal-Gly-BOC-(L-p-jodphenylalanyl)-Gly-BOC-*α*Nal-Gly-, F. 174° (Zers.) BOC-*β*Nal-Gly-BOC-Nle-Gly-BOC-(L-3-dekahydro-*α*Nal)-Gly-BOC-(L-homo-phenylalanyl)-Gly-BOC-Trp-Gly-(2-Propylamino-3-phenyl-propionyl)-Gly-, F. 60° (2-Butylamino-3-phenyl-propionyl)-Gly-, F. 129-131° (2-tert.-Butylamino-3-phenyl-propionyl)-Gly-(2-Morpholino-3-phenyl-propionyl)-Gly-(N-Isopropyl-carbamoyl)-Phe-Gly-, F. 193° (Zers.) (N-Isopentyl-carbamoyl)-Phe-Gly-, F. 189° (Zers.) (N,N-Dibutyl-carbamoyl)-Gly-(N-Benzyl-N-ethyl-carbamoyl)-Gly-(N-Benzyl-N-pentyl-carbamoyl)-Gly-(N-Benzyl-N-isopentyl-carbamoyl)-Gly-, F. 140-143° (N-Benzyl-N-4-methylpentyl-carbamoyl)-Gly-(N-Benzyl-N-3,3-dimethylbutyl-carbamoyl)-Gly-(N-Benzyl-N-3-ethoxypropyl-carbamoyl)-Gly-[N-Benzyl-N-3-(2-oxopyrrolidino)-propyl-carbamoyl]-Gly-[N-Benzyl-N-(3-morpholinopropyl)-carbamoyl|-Gly-Pyrrolidinocarbonyl-Phe-Gly-, F. 125-130° Piperidinocarbonyl-Phe-Gly-Morpholinocarbonyl-Phe-Sar-Piperidinoacetyl-Phe-Gly-, F. 145-147° Morpholinoacetyl-Nle-Gly-Morpholinoacetyl-Phe-Gly-, F. 154-156° Thiomorpholinoacetyl-Phe-Gly-, F. 162-164° S-Oxido-thiomorpholinoacetyl-Phe-Gly-, F. 175-176° S,S-Dioxido-thiomorpholinoacetyl-Phe-Gly-(3-Morpholino-propionyl)-Phe-Gly-(2-Methoxycarbonyl-pyrrolidino)-carbonyl-Phe-Gly-(2-tert.-Butoxycarbonyl-pyrrolidino)-carbonyl-Phe-Gly-, F. 123-125° (2-Benzyl-3-morpholinocarbonyl-propionyl)-(-)-(2-Benzyl-3-morpholinocarbonyl-propionyl)-(+)-(2-Benzyl-3-morpholinocarbonyl-propionyl)-3-(BOC-Phe-amino)-propionyl-, F. 199° 3-(Isopropoxycarbonyl-Phe-amino)-propionyl, F. 217° 3-(Morpholinoacetyl-Phe-amino)-propionyl-, F. 202-203° 3-(2-Benzyl-heptanoyl-amino)-propionyl-3-[2-(2-Pyridyl)-ethoxycarbonyl-Phe-amino)-propionyl-, F. 188° 4-(BOC-Phe-amino)-butyryl-4-(Isopropoxycarbonyl-Phe-amino)-butyryl-4-(Morpholinocarbonyl-Phe-amino)-butyryl-.

### Beispiel 14

Analog Beispiel 5 erhält man mit BOC-AHCP-OH das BOC-AHCP-Ile-(N-2-amino-5,6-dimethyl-3-pyrazinyl-methylamid), F. 175° (Zers.).

Analog erhält man BOC-AHCP-Ile-(N-4-amino-2-methyl-5-pyrimidinyl-methyl-amid), F. 223° BOC-AHCP-Ile-(N-2-pyridyl-methyl-amid), F. 142-144° BOC-AHCP-Ile-(N-3-pyridyl-methyl-amid), F. 162-163° BOC-AHCP-Ile-(N-4-pyridyl-methyl-amid), F. 166-168° 3-(BOC-Phe-amino)-propionyl-AHCP-Ile-NH₂, F. 223° [2-Benzyl-4-(2-pyridyl)-butyryl]-Gly-AHCP-Leu-NH₂, Öl 2-(2-Pyridyl)-ethoxycarbonyl-Phe-Gly-ACHP-Ile-NH₂, F. 103° (N-Benzyl-N-butyl-carbamoyl)-Gly-AHCP-Ile-OMe, F. 60° BOC-Phe-Gly-Sta-Ile-(N-4-amino-2-methyl-5-pyrimidinylmethyl-amid), F. 176-178° (N-Benzyl-N-isopentyl-carbamoyl)-Gly-Sta-Ile-(N-4-amino-2-methyl-5-pyrimidinyl-methyl-amid), F. 120° (Zers.) BOC-Phe-Gly-AHCP-Ile-(N-2,5,6-trimethyl-3-pyrazinylmethyl-amid),F. 96° (N-Benzyl-N-isopentyl-carbamoyl)-Gly-AHCP-Ile-(N-2-pyridylmethyl-amid), F. 66-71° (N-Benzyl-N-isopentyl-carbamoyl)-Gly-AHCP-Ile-(N-3-pyridylmethyl-amid), F. 72-76° (N-Benzyl-N-isopentyl-carbamoyl)-Gly-AHCP-Ile-(N-4-pyridylmethyl-amid), F. 75-79° (N-Benzyl-N-4-methylpentyl-carbamoyl)-Gly-AHCP-Ile-(N-3-pyridyl-methyl-amid) Morpholinocarbonyl-Phe-Gly-AHCP-Ile-(N-2-pyridyl-methyl-amid), F. 86-88° Morpholinocarbonyl-Phe-Gly-AHCP-Ile-(N-3-pyridyl-methyl-amid), F. 78° (Zers.) Morpholinocarbonyl-Phe-Gly-AHCP-Ile-(N-4-pyridyl-methyl-amid), F. 199-201° BOC-Phe-Gly-AHCP-Ile-(N-m-methyl-benzylamid) Isopropoxycarbonyl-Phe-Gly-AHCP-Ile-(N-m-aminomethyl-benzyl-amld),F. 142-146° (N-Benzyl-N-isopentyl-carbamoyl)-Gly-AHCP-Ile-(N-m-aminomethyl-benzyl-amid), F. 130-133° (N-Benzyl-N-4-methylpentyl-carbamoyl)-Gly-AHCP-Ile-(N-m-aminomethyl-benzyl-amid) BOC-Phe-Gly-AHCP-Ile-(N-3-piperidyl-methyl-amid), F. 168° (Zers.) BOC-Phe-Gly-AHCP-Ile-(N-4-piperidyl-methyl-amid), F. 140-146° BOC-Phe-Gly-AHCP-Ile-(N-4-piperidyl-amid), F. 142° BOC-Phe-Gly-AHCP-Ile-(N-1-benzyl-4-piperidyl-amid), F. 124° BOC-Phe-Gly-AHCP-Ile-[N-1-(2-hydroxyethyl)-4-piperidyl-amid],F. 114° Isopropoxycarbonyl-Phe-Gly-AHCP-Ile-(N-5-tetrazolyl-methyl-amid),F. 104-108° Morpholinocarbonyl-Phe-Gly-AHCP-Ile-(N-5-tetrazolyl-methyl-amid),F. 146° (Zers.) Morpholinocarbonyl-Phe-Gly-AHCP-[N-1-(2-amino-4-thiazolyl)-2-methyl-butyl-amid], F. 112° (Zers.) BOC-Phe-Gly-AHCP-Ile-(N-4-tetrahydropyranyl-amid), F. 124° BOC-Phe-Gly-AHCP-Ile-(N-4-methyl-5-imidazolyl-methyl-amid), F. 142° BOC-Phe-Gly-AHCP-D-Ile-(N-4-methyl-5-imidazolyl-methyl-amid),F. 175° 2-Isopropyl-4S-hydroxy-5S-(N-benzyl-N-isopentyl-carbamoyl)-Gly-amino-7-methyl-octanoyl-Ile-(N-2-pyridyl-methyl-amid), F. 172° Bis-(1-naphthylmethyl)-acetyl-Gly-AHCP-Ile-(N-m-aminomethyl-benzylamid) 3-[Bis-(1-naphthylmethyl)-acetyl-amino]-propionyl-AHCP-Ile-(N-m-aminomethyl-benzylamid) 3-[Bis-(1-naphthylmethyl)-acetyl-amino]-propionyl-Sta-Ile-(N-m-aminomethyl-benzylamid) 3-[Bis-(1-naphthylmethyl)-acetyl-amino]-propionyl-Sta-Ile-(N-4-amino-2-methyl-5-pyrimidinyl-methyl-amid).

### Beispiel 15

Analog Beispiel 3 erhält man aus BOC-Phe-Gly-OH und 2-Isopropyl-4S-hydroxy-5S-amino-6-cyclohexyl-hexansäure-(N-butylamid) das 2-Isopropyl-4S-hydroxy-5S-BOC-Phe-Gly-amino-6-cyclohexyl-hexansäure-(N-butylamid), F. 161-162°. (Diese Verbindung wird nicht beansprucht).

Analog erhält man die folgenden 2-Isopropyl-4S-hydroxy-6-cyclohexyl-hexanoyl-Ile-(N-4-amino-2-methyl-5-pyrimidinyl-methyl-amide): 5S-BOC-Phe-Gly-amino-5S-Isopropoxycarbonyl-Phe-Gly-amino-5S-Morpholinocarbonyl-Phe-Gly-amino-5S-Morpholinoacetyl-Phe-Gly-amino-5S-(2-Benzyl-heptanoyl)-Gly-amino-5S-(N-Benzyl-N-butyl-carbamoyl)-Gly-amino-5S-(N-Benzyl-N-isopentyl-carbamoyl)-Gly-amino-, F. 189-190° 5S-(2-Benzyl-4-oxo-5,5-dimethyl-hexanoyl)-Gly-amino-5S-(N-Benzyl-N-(3-moryholinopropyl)-carbamoyl]-Gly-aminosowie die folgenden 2-Isopropyl-4S-hydroxy-7-methyl-octanoyl-Ile-(N-4-amino-2-methyl-5-pyrimidinyl-methyl-amide): 5S-BOC-Phe-Gly-amino-5S-Isopropoxycarbonyl-Phe-Gly-amino-5S-Morpholinocarbonyl-Phe-Gly-amino-5S-Morpholinoacetyl-Phe-Gly-amino-5S-(2-Benzyl-heptanoyl)-Gly-amino-5S-(N-Benzyl-N-butyl-carbamoyl)-Gly-amino-5S-(N-Benzyl-N-isopentyl-carbamoyl)-Gly-amino-5S-(2-Benzyl-4-oxo-5,5-dimethyl-hexanoyl)-Gly-amino-5S-[N-Benzyl-N-(3-morpholinopropyl)-carbamoyl]-Gly-amino-5S-[3-(Morpholinoacetyl-Phe-amino)-propionyl-amino]-5S-(3-BOC-Phe-propionyl-amino)-5S-[3-(Morpholinoacetyl-Phe)-propionyl-amino]-5S-(3-BOC-Phe-amino-propionyl-amino)-5S-(3-Thia-4-BOC-Phe-butyryl-amino)-5S-[3-Thia-4-(morpholinoacetyl-Phe)-butyryl-amino]-.

### Beispiel 16

Analog Beispiel 4 erhält man aus BOC-Phe-Gly-OH und H-AHCP-Leu-N-3-dimethylamino-propyl-amid das BOC-Phe-Gly-AHCP-Leu-N-(3-dimethylamino-propyl-amid), F. 118-119°. (Diese Verbindung wird nicht beansprucht).

Analog erhält man die nachstehenden AHCP-Ile-(N-4-amino-2-methyl-5-pyrimidinyl-methyl-amide): BOC-Pro-Phe-Gly-, F. 142-143° Isovaleryl-Phe-Gly-, F. 194-195° 3,3-Dimethyl-butyryl-Phe-Gly-, F. 142-143° 4-(BOC-Phe)-3-thia-butyryl-, F. 130° 3-(BOC-Phe)-propionyl-, F. 135° 3-(2-Pyridyl)-propoxy-carbonyl-Phe-Gly-, F. 114° 3-(4-Pyridyl)-propoxy-carbonyl-Phe-Gly, F. 131° [2S-(1-Naphthylmethyl)-3-(N-3-morpholinopropyl-carbamoyl)-propionyl]-Gly-, F. 124° [2R-(1-Naphthylmethyl)-3-(N-3-morpholinopropyl-carbamoyl)-propionyl]-Gly-, F. 125° Bis-(1-naphthylmethyl)-acetyl-Gly-3-[Bis-(1-naphthylmethyl)-acetyl-amino]-propionyl-.

Die nachstehenden Beispiele betreffen pharmazeutische Zubereitungen.

### Beispiel A: Injektionsgläser

Eine Lösung von 100 g (N-Benzyl-N-2-phenylethyl-carbamoyl)-Gly-AHCP-Ile-(N-2-amino-5,6-dimethyl-3-pyrazinyl-methyl-amid) und 5 g Dinatriumhydrogenphosphat in 3 I zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 500 mg Wirkstoff.

### Beispiel B: Suppositorien

Man schmilzt ein Gemisch von 500 g [2-Benzyl-4-(2-oxo-pyrrolidino)-butyryl]-Gly-AHCP-Ile-(N-2-amino-5,6-dimethyl-3-pyrazinyl-methyl-amid) mit 100 g Sojalecithin und 1400 g Kakaobutter, gießt in Formen und läßt erkalten. Jedes Suppositorium enthält 500 mg Wirkstoff.

## Patentansprüche

1. Aminosäurederivate der Formel I
X-(NR¹-CHR²-CO)ₖ-Z-(CH₂)ₘ-CO-NR³-CHR⁴-CR⁵-(CHR⁶)ₙ-CO-E-Q-Y I
worin
X H, R⁷⁻O-CₘH₂ₘ-CO-, R⁷-CₘH₂ₘ-O-CO-, R⁷-CₘH₂ₘ-CO-, (R⁷-CₘH₂ₘ)-L(R⁸-CₚH₂ₚ)-CᵣH₂ᵣ-CO-,
oder (R⁷-CₘH₂ₘ-(T)ₓ-CO-CᵣH₂ᵣ)-L(R⁸-CₚH₂ₚ)-CO-,
Z -CH₂-, -NR⁹- oder -CH₂-S-,
E
1 oder 2 peptidartig miteinander verbundene Aminosäurereste ausgewählt aus der Gruppe bestehend aus Ile, Leu, Phe und Val,
T
O oder NH,
Q-Y
OH, OA, NH-CₜH₂ₜ-R¹⁰ oder NHC_{w}H_{2w}-(CR¹¹)ₛ-CₜH₂ₜ-R¹⁰,
R¹ und R⁹
jeweils H oder A,
R²
A, Ar-alkyl, Cyclohexylmethyl, 1- oder 2-Dekalylmethyl oder Het-alkyl,
R³
H,
R⁴
A, Cycloalkylalkyl mit 4-11 C-Atomen oder Benzyl,
R⁵
(H,OH) oder (H, NH₂),
-(CHR⁶)ₙ-
-CH²-, -CH₂CH₂- oder -CH₂CHA-,
R⁷ und R¹⁰
jeweils H, A, Ar oder Het,
R⁸
Benzyl oder 1- oder 2- Naphthylmethyl,
R¹¹
(H,OH), (H,NH₂) oder =O,
L
CH oder N,
k und x
jeweils 0 oder 1,
m
1,2 oder 3,
p, r und t
jeweils 0, 1, 2, 3, 4 oder 5,
s
1 oder 2,
w
1, 2, 3, 4 oder 5,
Ar
unsubstituiertes oder einfach durch A, AO, Hal oder Aminoalkyl mit 1-8 C-Atomen substituiertes Phenyl oder unsubstituiertes Naphthyl,
Het
einen gesättigten oder ungesättigten 5-oder 6-gliedrigen heterocyclischen Rest mit 1-4 N-, O- und/oder S-Atomen, der mit einem Benzolring kondensiert und/oder ein- oder mehrfach durch A, HO, Carbonylsauerstoff, H₂N, AOOC, H₂NCO, Hydroxyalkyl und/oder Aminoalkyl mit jeweils 1-8 C-Atomen substituiert sein kann und/oder dessen S-Heteroatom auch oxydiert sein kann,
Hal
F, Cl, Br oder J und
A Alkyl mit 1-8 C-Atomen bedeuten,
sowie deren Salze.

2. a) (N-Benzyl-N-2-phenylethylcarbamoyl)-Gly-AHCP-Ile-(N-2-amino-5,6-dimethyl-3-pyrazinylmethyl-amid);
b) [2-Benzyl-4-(2-oxopyrrolidino)-butyryl]-Gly-AHCP-Ile-(N-2-amino-5,6-dimethyl-3-pyrazinylmethyl-amid);
c) (2-Benzyl-heptanoyl)-Gly-AHCP-Ile-(N-4-amino-2-methyl-5-pyrimidinyl-methyl-amid);
d) (+)-[2-(1-Naphthylmethyl)-3-(N-2-(2-pyridyl)-ethyl-carbamoyl)-propionyl]-Gly-AHCP-Ile-(N-4-amino-2-methyl-5-pyrimidinyl-methyl-amid);
e) (N-Benzyl-N-isopentyl-carbamoyl)-Gly-AHCP-Ile-(N-4-amino-2-methyl-5-pyrimidinyl-methyl-amid);
f) BOC-Phe-Gly-AHCP-Ile-(N-3-pyridylmethyl-amid);
g) BOC-Phe-Gly-AHCP-Ile-NH₂;
h) BOC-Phe-Gly-AHCP-Ile-(N-2-amino-5,6-dimethyl-3-pyrazinyl-methyl-amid);
i) BOC-Phe-Gly-AHCP-Ile-(N-4-amino-2-methyl5-pyrimidinyl-methyl-amid);
j) Morpholinoacetyl-Phe-Gly-AHCP-Ile-(N-2-amino-5,6-dimethyl-3-pyrazinyl-methyl-amid).

3. Verfahren zur Herstellung eines Aminosäurederivats der Formel I sowie von seinen Salzen, **dadurch gekennzeichnet, daß** man es aus einem seiner funktionellen Derivate durch Behandeln mit einem solvolysierenden oder hydrogenolysierenden Mittel in Freiheit setzt oder daß man eine Carbonsäure der Formel II
X-(NR¹-CHR²-CO)ₖ-G¹-OH II
worin
G¹
(a) fehlt,
(b) -Z-(CH₂)ₘ-CO-,
(c) -Z-(CH₂)ₘ-CO-W-,
(d) -Z-(CH₂)ₘ-CO-W-E¹-,
(e) -Z-(CH₂)ₘ-CO-W-E- und
W -NR³-CHR⁴-CR⁵-(CHR⁶)ₙ-CO- bedeuten
oder eines ihrer reaktionsfähigen Derivate mit einer Verbindung der Formel III
H-G² III
worin
G²
(a) -Z¹-(CH₂)ₘ-CO-W-E-Q-R
(b) -W-E-Q-Y,
(c) -E-Q-Y,
(d) -E²-Q-Y,
(e) -Q-Y,
Z¹ -O- oder -NR⁹- und
E¹ + E² zusammen E bedeuten
oder einem ihrer reaktionsfähigen Derivate umsetzt,
und/oder daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Amino- oder Hydroxygruppe acyliert und/oder eine Ketogruppe zu einer CHOH-Gruppe reduziert oder zu einer CH(NH₂)-Gruppe reduktiv aminiert und/oder eine Verbindung der Formel I durch Behandeln mit einer Säure in eines ihrer Salze überfuhrt.

4. Verfahren zur Herstellung pharmazeutischer Zubereitungen, **dadurch gekennzeichnet, daß** man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, **gekennzeichnet durch** einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels zur Bekämpfung der reninabhängigen Hypertension oder des Hyperaldosteronismus.

## Claims

1. Amino acid derivatives of the formula I
X-(NR¹-CHR²-CO)ₖ-Z-(CH₂)ₘ-CO-NR³-CHR⁴-CR⁵-(CHR⁶)ₙ-CO-E-Q-Y I
in which
X
is H, R⁷-O-CₘH₂ₘ-CO-, R⁷-CₘH₂ₘ-O-CO-, R⁷-CₘH₂ₘ-CO-, (R⁷-CₘH₂ₘ)-L(R⁸-CₚH₂ₚ)-CᵣH₂ᵣ-CO- or (R⁷-CₘH₂ₘ- (T)ₓ-CO-CᵣH₂ᵣ)-L-(R⁸-CₚH₂ₚ)-CO-,
Z
is -CH₂-, -NR⁹- or -CH₂-S-,
E
is 1 or 2 amino acid residues which are bonded together in the manner of a peptide and are selected from the group comprising Ile, Leu, Phe and Val,
T
is O or NH,
Q-Y
is OH, OA, NH-CₜH₂ₜ-R¹⁰ or NH-C_{w}H_{2w}-(CR¹¹)ₛ-CₜH₂ₜ-R¹⁰,
R¹ and R⁹
are each H or A,
R²
is A, Ar-alkyl, cyclohexylmethyl, 1- or 2-decalylmethyl or Het-alkyl,
R³
is H,
R⁴
is A, cycloalkylalkyl with 4-11 C atoms or benzyl,
R⁵
is (H, OH) or (H, NH₂),
-(CHR⁶)ₙ-
is -CH₂-, -CH₂CH₂- or -CH₂CHA-,
R⁷ and R¹⁰
are each H, A, Ar or Het,
R⁸
is benzyl or 1- or 2-naphthylmethyi,
R¹¹
is (H, OH), (H, NH₂) or =O,
L
is CH or N,
k and x
are each 0 or 1,
m
is 1, 2 or 3,
p, r and t
are each 0, 1, 2, 3, 4 or 5,
s
is 1 or 2,
w
is 1, 2, 3, 4 or 5,
Ar
is phenyl which is unsubstituted or substituted once by A, AO, Hal or aminoalkyl having 1-8 C atoms or is unsubstituted naphthyl,
Het
is a saturated or unsaturated 5- or 6-membered heterocyclic radical which has 1-4 N, O and/or S atoms, which can be fused to a benzene ring and/or substituted once or several times by A, HO, carbonyl oxygen, H₂N, AOOC, H₂NCO, hydroxyalkyl and/or aminoalkyl, each having 1-8 C atoms, and/or whose S heteroatom can also be oxidized,
Hal
is F, Cl, Br or I and
A
is alkyl having 1-8 C atoms,
and their salts.

2. a) (N-Benzyl-N-2-phenylethylcarbamoyl)-Gly-AHCP-Ile-(N-2-amino-5,6-dimethyl-3-pyrazinylmethylamide);
b) [2-benzyl-4-(2-oxopyrrolidino)-butyryl]-Gly-AHCP-Ile-(N-2-amino-5,6-dimethyl-3-pyrazinylmethyl-amide);
c) (2-benzyl-heptanoyl)-Gly-AHCP-Ile-(N-4-amino-2-methyl-5-pyrimidinylmethylamide);
d) (+)-[2-(1-naphthylmethyl)-3-(N-2-(2-pyridyl)-ethyl-carbamoyl)-propionyl]-Gly-AHCP-Ile-(N-4-amino-2-methyl-5-pyrimidinyl-methyl-amide);
e) (N-benzyl-N-isopentyl-carbamoyl)-Gly-AHCP-Ile-(N-4-amino-2-methyl-5-pyrimidinyl-methyl-amide);
f) BOC-Phe-Gly-AHCP-Ile-(N-3-pyridyl-methyl-amide);
g) BOC-Phe-Gly-AHCP-Ile-NH₂;
h) BOC-Phe-Gly-AHCP-Ile-(N-2-amino-5,6-dimethyl-3-pyrazinyl-methyl-amide);
i) BOC-Phe-Gly-AHCP-Ile-(N-4-amino-2-methyl-5-pyrimidinyl-methyl-amide);
j) morpholinoacetyl-Phe-Gly-AHCP-Ile-(N-2-amino-5,6-dimethyl-3-pyrazinyl-methyl-amide).

3. Process for the preparation of an amino acid derivative of the formula I, and of its salts, **characterized in that** it is liberated from one of its functional derivatives by treatment with a solvolysing or hydrogenolysing agent,
or **in that** a carboxylic acid of the formula II
X-(NR¹-CHR²-CO)ₖ-G¹-OH II
in which
G¹ is
(a) absent
(b) -Z-(CH₂)ₘ-CO-,
(c) -Z-(CH₂)ₘ-CO-W-,
(d) -Z-(CH₂)ₘ-CO-W-E¹-,
(e) -Z-(CH₂)ₘ-CO-W-E- and
W is -NR³-CHR⁴-CR⁵-(CHR⁶)ₙ-CO- or one of its reactive derivatives,
is reacted with a compound of the formula III
H-G² III
in which
G2 is
(a) -Z¹-(CH₂)ₘ-CO-W-E-Q-Y,
(b) -W-E-Q-Y,
(c) -E-Q-Y,
(d) -E²-Q-Y,
(e) -Q-Y,
Z¹ is -O- or -NR⁹- and
E¹ + E² are together E,
or one of its reactive derivatives,
and/or **in that**, where appropriate, in a compound of the formula I a functionally modified amino and/or hydroxyl group is liberated by treatment with solvolysing or hydrogenolysing agents, and/or a free amino or hydroxyl group is acylated, and/or a keto group is reduced to a CHOH group or is reductively aminated to a CH(NH₂) group, and/or a compound of the formula I is converted into one of its salts by treatment with an acid.

4. Process for the preparation of pharmaceutical formulations, **characterized in that** a compound of the formula I, and/or one of its physiologically acceptable salts, is converted into a suitable dosage form together with at least one solid, liquid or semi-liquid vehicle or auxiliary and, where appropriate, in combination with one or more other active compound(s).

5. Pharmaceutical formulation, **characterized by** containing at least one compound of the formula I and/or one of its physiologically acceptable salts.

6. Use of compounds of the formula I, or of their physiologically acceptable salts, for the preparation of a medicament for controlling renindependent hypertension or hyperaldosteronism.

## Revendications

1. Dérivés d'acides aminés de formule I
X-(NR¹-CH²-CO)ₖ-Z-(CH₂)ₘ-CO-NR³-CHR⁴-CR⁵-(CHR⁶)ₙ-CO-E-Q-Y
dans laquelle
X
représente H, R⁷-O-CₘH₂ₘ-CO, R⁷-CₘH₂ₘ-O-CO-, R⁷-CₘH₂ₘ-CO-, (R⁷-CₘH₂ₘ)-L(R⁸-CₚH₂ₚ)-CᵣH₂ᵣ-CO-, ou (R⁷-CₘH₂ₘ-(T)ₓ-CO-CᵣH₂ᵣ)-L(R⁸ -CₚH₂ₚ)-CO-,
Z
représente-CH₂-, -NR⁹- ou -CH₂-S-, E
représente 1 ou 2 radicaux acides aminés reliés entre eux par une liaison peptidique choisis dans le groupe constitué par Ile, Leu, Phe et Val,
T
représente O ou NH,
Q-Y
représente OH, OA, NH-CₜH₂ₜ-R¹⁰ OU NH-C_{w}H_{2w}-(CR¹¹)ₛ-CₜH₂ₜ-R¹⁰,
R¹ et R⁹
représentent chacun H ou A,
R²
représente A, Ar-alkyle, cyclohexylméthyle,
1 ou 2-décalylméthyle ou Het-alkyle, R³
représente H,
R⁴
représente A, un cycloalkyle en C₄ à C₁₁ ou un benzyle,
R⁵
représente (H,OH) ou (H, NH₂), -(CHR⁶)ₙ-
représente -CH₂-, -CH₂CH₂- ou -CH₂CHA-, R⁷ et R¹⁰
représentent chacun H, A, Ar ou Het,
R⁸
représente un benzyle ou 1 ou 2-naphtylméthyle,
R¹¹
représente (H,OH), (H,NH₂) ou =O,
L
représente CH ou N,
k et x
valent chacun 0 ou 1,
m
vaut 1, 2 ou 3
p, r et t
valent chacun 0, 1, 2, 3, 4 ou 5,
s
vaut 1 ou 2,
w
vaut 1, 2, 3, 4 ou 5.
Ar
représente un phényle non suostitué ou monosubstitué par A, AO, Hal ou un aminoalkyle en C₁ à C₈, ou un naphtyle non substitué,
Het
représente un radical hétérocyclique à 5 ou 6 chaînes non saturées ou insaturées avec de 1 à 4 atomes de N, O et/ou S, qui pent être condensé avec un noyau benzène et/ou peut être mono ou polysubstitué par A, HO, un oxygène carbonylique, H₂N, AOOC, H₂NCO, un hydroxyalkyle et/ou un aminoalkyle avec à chaque fois de 1 à 8 atomes de carbone, et/ou dont l'hétéroatome s peut également être oxydé,
Hal
représente F, Cl, Br ou J,
et
A représente un alkyle en C₁ à C₈, ainsi que leurs sels.

2. a) (N-benzyl-N-2-phényléthyl-carbamoyl)-Gly-AHCP-Ile-(N-2-amino-5,6-diméthyl-3-pyrazinyl-méthyl-amide);
b) [2-benzyl-4-(2-oxopyrrolidono)butyryl]-Gly-AHCP-Ile-(N-2-amino-5,6-diméthyl-3-pyrazinyl-méthyl-amide);
c) (2-benzyl-heptanoyl)-Gly-AHCP-Ile-(N-4-amino-2-méthyl-5-pyrimidinyl-méthyl-amide);
d) (+)-[2-(1-naphtylméthyl)-3-(N-2-(2-pyridyl)-éthylcarbamoyl)-propionyl]-Gly-AHCP-Ile-(N-4-amino-2-méthyl-5-pyrimidinyl-méthyl-amide);
e) (N-benzyl-N-isopentyl-carbamoyl)-Gly-AHCP-Ile-(N-4-amino-2-méthyl-5-pyrimidinyl-méthyl-amide);
f) BOC-Phe-Gly-AHCP-Ile-(N-3-pyridylméthyl-amide);
g) BOC-Phe-Gly-AHCP-Ile-NH₂;
h) BOC-Phe-Gly-AHCP-Ile-(N-2-amino-5,6-diméthyl-3-pyrazinyl-méthyl-amide);
i) BOC-Phe-Gly-AHCP-Ile-(N-4-amino-2-méthyl-5-pyrimidinyl-méthyl-amide);
j) Morpholinoacétyl-Phe-Gly-AHCP-Ile-(N-2-amino-5,6-diméthyl-3-pyrazinyl-méthyl-amide)

3. Procédé de préparation d'un dérivé d'acides aminés de formule I ainsi que de ses sels, **caractérisé en ce qu'**on le libère à partir de ces dérivés fonctionnels par traitememt avec un agent de solvolyse ou d'hydrogénolyse,
ou **en ce qu'**on fait réagir un acide carboxylique de formule II
X-(NR¹-CHR²-CO)ₖ-G¹-OH II
dans laquelle G¹
(a) manque,
(b) représente -Z-(CH₂)ₘ-CO-,
(c) représente -Z-(CH₂)ₘ-CO-W-,
(d) -Z-(CH₂)ₘ-CO-W-E¹-,
(e) -Z-(CH₂)ₘ-CO-W-E- et
W représente -NR³-CHR⁴-CR⁵-(CHR⁶)ₙ-CO- ou un de ses dérivés réactifs avec un composé de formule II
H-G² III
dans laquelle G²
(a) représente -Z¹-(CH₂)ₘ-CO-W-E-Q-R
(b) -W-E-Q-Y,
(c) -E-Q-Y,
(d) -E²-Q-Y,
(e) -Q-Y-,
Z¹ représente -O- ou -NR⁹- et
E¹ + E² représentent ensemble E;
ou un de ses dérivés réactifs,
et/ou **en ce que** le cas échéant, on libère dans un composé de formule I un groupe amino et/ou hydroxy fonctionnellement modifié par traitement avec des agents de solvolyse ou d'hydrogénolyse et/ou **en ce qu'**on acyle un groupe amino ou hydroxy libre et/ou **en ce qu'**on réduit un groupe céto CHOH ou **en ce qu'**on lamine de façon réductrice en un groupe CH(NH₂) et ou **en ce qu'**on transforme un composé de formule I en un de ses sels par traitement avec un acide.

4. Procédé de préparations pharmaceutiques, **caractérisé en ce qu'**on met sous une forme posologique approprié un composé de formule I et/ou un de ses sels physiologiquement acceptables avec au moins un support ou additif solide, liquide ou semi-liquide et le cas échéant en combinaison avec une ou plusieurs autres substances actives.

5. Préparation pharmaceutique **caractérisée en ce qu'**elle contient au moins un composé de formule I et/ou un de ses sels physiologiquement acceptables.

6. Application de composés de formule I ou de leurs sels Physiologiquement acceptables à la préparation d'un médicament pour combattre l'hypertension réninodépendante ou l'hyperaldostéronisme.
